# EUROPEAN PATENT APPLICATION

(11) **EP 3 786 174 A1**
(43) Date of publication of application: **03.03.2021**
(21) Application number: 19193906.5
(22) Date of filing: 27.08.2019
(51) Int. Cl.: C07K 1/36, C07K 16/00

(54) **METHODS FOR ANTIBODY PURIFICATION**

(71) Applicant: Ichnos Sciences SA, 2300 La Chaux-de-Fonds (CH)
(72) Inventor: Duarte, Lionel, 2300 La Chaux-de-Fonds (CH); Cahuzac, Laure, 2300 La Chaux-de-Fonds (CH); GIOVANNINI, Roberto, 2300 La Chaux-de-Fonds (CH); LAMBERCIER, Monia, 2300 La Chaux-de-Fonds (CH)

(57) **Abstract**

The present invention relates to a method for preparing a purified therapeutic recombinant antibody solution from a clarified cell harvest. In particular, the method of the present invention allows the efficient removal of impurities, such as host cell proteins, and the recovery of high yields of the therapeutic recombinant antibody.

## Description

### TECHNICAL FILED

The present invention relates to a method for preparing a purified therapeutic recombinant antibody solution from a clarified cell harvest. In particular, the method of the present invention allows the efficient removal of impurities, such as host cell proteins, and the recovery of high yields of the therapeutic recombinant antibody.

### BACKGROUND

The increasing use of proteins, such as antibodies, as pharmaceuticals for clinical applications, has made the development of high efficient purification methods crucial for their manufactory. Typically, therapeutic proteins are produced by cells, using for instance mammalian or bacterial cells engineered so as to express the protein of interest and cultured under controlled conditions that aid their growth and the expression of the protein of interest. Optimal culturing conditions are obtained by the control and adjustment of factors such as the formulation of the cell culture medium, the nutrient supply modality and the culturing time.

The cell culture is a complex mix comprising, besides the host cells expressing the protein of interest and the protein of interest itself, cell debris, for instance generated upon cell death, colloidal particles, such as DNA, RNA and host cell proteins (HCP), and other (bio)molecules secreted by the cultured cells. Separating the protein of interest from this mix is not a simple operation especially considering the increased efficiency of the upstream processes that lead to higher titers of the biomolecule of interest at the end of the cell culture.

As a consequence, the recovery and purification of a protein of interest requires multiple steps. Generally, an initial step of cell culture clarification is used to remove residual biomass; the clarified cell culture fluid is then subjected to steps of chromatography, filtration and viral inactivation to remove the remaining impurities such as host cell proteins, product variants, host cell DNA, small molecules, process related contaminants, endotoxins and viral particles.

Optimizing the steps and the number of operations to perform during a purification is crucial to reduce the time and the costs of the process, while keeping its yield high. Additionally, to ensure the safety of a protein to use as a therapeutic, it is required that the protein obtained at the end of the purification process is highly pure. Nevertheless establishing an efficient purification processes able to remove impurities, such as HCP and DNA, and at the same time, to provide high yields of the molecule of interest remains challenging.

### SUMMARY

The present invention relates to a method for preparing a purified therapeutic recombinant antibody solution from a clarified cell harvest. In particular, the method according to the present invention allows the efficient removal of impurities, such as host cell proteins, and the recovery of high yields of the therapeutic recombinant antibody.

An established purification process, also referred herein to as reference purification process (RPP), has been used for the purification of recombinant therapeutic antibodies (such as Ab1 and Ab2 described in Examples 1 to 5).

In particular, RPP is characterized by the steps of:
- Affinity chromatography;
- Virus inactivation;
- Cation exchange chromatography;
- Ultrafiltration/Diafiltration (UF/DF1);
- Anion exchange chromatography;
- Virus filtration;
- Ultrafiltration/Diafiltration (UF/DF2).

RPP comprises a UF/DF1 step which is expensive and time-consuming. UF/DF1 permits to concentrate the product and change the buffer to make it suitable for the transition of the solution from the preceding cation exchange chromatography to the following anion exchange chromatography, therefore the removal of this step is not trivial. In fact, the buffer used for cation exchange chromatography are incompatible with the ones used for anion exchange chromatography and vice versa. Also, since UF/DF1 aids impurity reduction, without UF/DF1 the two ion exchange chromatography steps would be more challenged in terms of impurities removal, and difficulties coluld arise in the way they would fit the conditions of the other steps. Nevertheless, the inventors of the method according to the present invention have developed two new purification processes, referred herein to as purification process 1 (PP1) and purification process 2 (PP2), without UF/DF1.

PP1 is characterized by the steps of:
- Affinity chromatography;
- Virus inactivation;
- Cation exchange chromatography;
- pH adjustment;
- Anion exchange chromatography;
- Virus filtration;
- Ultrafiltration/Diafiltration.

PP2 is characterized by the steps of:
- Affinity chromatography;
- Virus inactivation;
- Anion exchange chromatography;
- pH adjustment;
- Cation exchange chromatography;
- Virus filtration;
- Ultrafiltration/Diafiltration.

In the newly developed processes, the removal of UF/DF1 step allows reducing the complexity of the process and consequently decreasing time and costs of the process as well as decreasing the possibility of manipulation errors. Additionally, in the present invention not just the complexity of the reference purification process has been reduced but also the performances of the developed processes which lead to a higher yields and higher purity removal, such as higher host cell protein removal.

The present invention relates to a method for preparing a purified therapeutic recombinant antibody solution from a clarified cell harvest, characterized by subjecting said clarified cell harvest to (i) an affinity chromatography step, (ii) a viral inactivation step, (iii) at least two steps of ion exchange chromatography, wherein said purified therapeutic recombinant antibody solution comprises less than about 40 ppm of host cell proteins and wherein the yield of said at least two steps of ion exchange chromatography is higher than about 70%.

In one aspect, the affinity chromatography used in the disclosed method is protein A affinity chromatography.

In another aspect, viral inactivation according to the method disclosed herein is performed on the protein A chromatography eluate and the resulting viral inactivated solution undergoes the first of the at least two steps of ion exchange chromatography.

In a further aspect, after the first ion exchange chromatography step, the pH of the resulting ion exchange chromatography eluate is adjusted so as to be the same as the elution buffer of the second ion exchange chromatography step.

According to the present invention, the at least two steps of ion exchange chromatography comprise a first step of cation exchange chromatography and a second step of anion exchange chromatography; or the at least two steps of ion exchange chromatography comprise a first step of anion exchange chromatography and a second step of cation exchange chromatography.

In one aspect of the present invention, cation exchange chromatography is carried out in bind-eluate mode wherein said protein is eluted with an elution buffer comprising between about 20 mM and about 30 mM of Citrate, and between about 50 mM and about 120 mM of NaCl at pH between about 5 and about 6.

In another aspect of the present invention, anion exchange chromatography is carried out by membrane absorption in flow-through mode using between about 20 mM and 60 mM of Histidine or Tris buffer at a pH between about 6.5 and about 8.

In a more particular aspect, membrane absorption is carried out using about 50 mM Histidine at a pH between about 7 and about 7.5 at a flow rate comprised between about 2 MV/min and about 30 MV/min, and with a loading factor comprised between about 1 g/mL and about 15 g/mL

In an even more particular aspect, membrane absorption is carried out using about 50 mM Histidine at a pH selected from the group comprising about 7, about 7.2 and about 7.4, at a flow rate selected from the group comprising about 5 MV/min and about 25 MV/min, with a loading factor selected from the group comprising about 2.5 g/mL, about 5 g/mL, about 7.5 g/mL and about 10 g/mL.

In a specific embodiment, membrane absorption is carried out using about 50 mM Histidine at a pH 7.2, at a flow rate of about 25 MV/min, with a loading factor of about 10 g/mL.

In a further aspect of the current invention, cation exchange chromatography is carried out using an elution buffer comprising about 25 mM of Citrate and about 100 mM or 60 mM of NaCl.

In certain embodiments of the present invention, the purified therapeutic recombinant antibody solution resulting from the disclosed method comprises less than about 20 ppm of host cell proteins and/or less than about 0.8 pg/mg DNA.

According to the current disclosure, the protein titer of the clarified cell harvest is comprised between about 0.5 g/L and about 10 g/L.

Also disclosed by the present invention is a process for preparing a purified therapeutic recombinant antibody solution from a clarified cell harvest, comprising the steps of:
(i) Protein A chromatography of a clarified cell harvested antibody material;
(ii) Incubation of the resulting PA eluate at pH 3.5;
(iii) Neutralization of the resulting viral inactivated solution to pH7.2, followed by 0.2 µm filtration;
(iv) Anion exchange chromatography of the neutralized viral inactivated solution according to claim 11, followed by 0.2 µm filtration;
(v) Cation exchange chromatography of the anion exchange chromatography eluate according to claim12, followed by 0.2 µm filtration.

The current invention also relates to a process for the production of a bulk drug substance comprising the steps of:
(i) Protein A chromatography of a clarified cell harvested antibody material;
(ii) Incubation of the resulting PA eluate at pH 3.5;
(iii) Neutralization of the resulting viral inactivated solution to pH7.2, followed by 0.2 µm filtration;
(iv) Anion exchange chromatography of the neutralized viral inactivated solution according to claim 11, followed by 0.2 µm filtration;
(v) Cation exchange chromatography of the anion exchange chromatography eluate according to claim12, followed by 0.2 µm filtration;
(vi) Virus nanofiltration;
(vii) Concentration of the product by ultrafiltration and continuous diafiltration into preformulation buffer, followed by 0.2 um filtration;
(viii) Excipient addition;
(ix) 0.2 µm filtration.

The present invention also relates to the purified therapeutic recombinant antibody solution or the bulk drug substance obtained by said processes.

As used herein, the following terms have the following meanings: "a", "an", and "the" as used herein refers to both singular and plural unless the context clearly dictates otherwise. Unless otherwise defined, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures utilized in connection with, and techniques of cell and tissue culture, molecular biology, and protein and oligo- or polynucleotide chemistry, laboratory procedures and techniques of analytical chemistry, synthetic organic chemistry, and medicinal and pharmaceutical chemistry described herein are those well-known and commonly used in the art.

The term "antibody" as referred to herein includes whole antibodies and any antibody fragment, such as any antigen binding fragments or single chains thereof. An "antibody" refers to a glycoprotein comprising at least two heavy (H) chains and two light (L) chains inter-connected by disulfide bonds, or an antigen binding fragment thereof. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR) with are hypervariable in sequence and/or involved in antigen recognition and/or usually form structurally defined loops, interspersed with regions that are more conserved, termed framework regions (FR or FW). Each VH and VL is composed of three CDRs and four FWs, arranged from amino- terminus to carboxy-terminus in the following order: FW1, CDR1, FW2, CDR2, FW3, CDR3, FW4. The amino acid sequences of FW1, FW2, FW3, and FW4 all together constitute the "non-CDR region" or "non-extended CDR region" of VH or VL as referred to herein.

The term "heavy chain variable framework region" as referred herein may comprise one or more (e.g., one, two, three and/or four) heavy chain framework region sequences (e.g., framework 1 (FW1), framework 2 (FW2), framework 3 (FW3) and/or framework 4 (FW4)). Preferably the heavy chain variable region framework comprises FW1, FW2 and/or FW3, more preferably FW1, FW2 and FW3. The term "light chain variable framework region" as referred herein may comprise one or more (e.g., one, two, three and/or four) light chain framework region sequences (e.g., framework 1 (FW1), framework 2 (FW2), framework 3 (FW3) and/or framework 4 (FW4)). Preferably the light chain variable region framework comprises FW1, FW2 and/or FW3, more preferably FW1, FW2 and FW3. The variable regions of the heavy and light chains contain a binding domain that interacts with an antigen. The constant regions of the antibodies may mediate the binding of the immunoglobulin to host tissues or factors, including various cells of the immune system (e.g., effector cells) and the First component (C1q) of the classical complement system. Antibodies are grouped into classes, also referred to as isotypes, as determined genetically by the constant region. Human constant light chains are classified as kappa (CK) and lambda (CX) light chains. Heavy chains are classified as mu (µ), delta (δ), gamma (γ), alpha (a), or epsilon (ε), and define the antibody's isotype as IgM, IgD, IgG, IgA, and IgE, respectively. Thus, "isotype" as used herein is meant any of the classes and/or subclasses of immunoglobulins defined by the chemical and antigenic characteristics of their constant regions. The known human immunoglobulin isotypes are IgG1 (IGHG1), IgG2 (IGHG2), IgG3 (IGHG3), IgG4 (IGHG4), IgA1 (IGHA1), IgA2 (IGHA2), IgM (IGHM), IgD (IGHD), and IgE (IGHE). The so-called human immunoglobulin pseudo-gamma IGHGP gene represents an additional human immunoglobulin heavy constant region gene which has been sequenced but does not encode a protein due to an altered switch region (Bensmana M et al., (1988) Nucleic Acids Res. 16(7): 3108). In spite of having an altered switch region, the human immunoglobulin pseudo-gamma IGHGP gene has open reading frames for all heavy constant domains (CH1 -CH3) and hinge. All open reading frames for its heavy constant domains encode protein domains which align well with all human immunoglobulin constant domains with the predicted structural features. This additional pseudo-gamma isotype is referred herein as IgGP or IGHGP. Other pseudo immunoglobulin genes have been reported such as the human immunoglobulin heavy constant domain epsilon PI and P2 pseudo-genes (IGHEP1 and IGHEP2). The IgG class is the most commonly used for therapeutic purposes. In humans this class comprises subclasses IgG1, IgG2, IgG3 and IgG4. In mice this class comprises subclasses IgG1, IgG2a, IgG2b, IgG2c and IgG3.

Antibody fragments include, but are not limited to, (i) the Fab fragment consisting of VL, VH, CL and CHI domains, including Fab' and Fab'-SH, (ii) the Fd fragment consisting of the VH and CHI domains, (iii) the Fv fragment consisting of the VL and VH domains of a single antibody; (iv) the dAb fragment (Ward ES et al., (1989) Nature, 341 : 544-546) which consists of a single variable, (v) F(ab')2 fragments, a bivalent fragment comprising two linked Fab fragments (vi) single chain Fv molecules (scFv), wherein a VH domain and a VL domain are linked by a peptide linker which allows the two domains to associate to form an antigen binding site (Bird RE et al, (1988) Science 242: 423-426; Huston JS et al, (1988) Proc. Natl. Acad. Sci. USA, 85: 5879-83), (vii) bispecific single chain Fv dimers (PCT/US92/09965), (viii) "diabodies" or "triabodies", multivalent or multispecific fragments constructed by gene fusion (Tomlinson I & Hollinger P (2000) Methods Enzymol. 326: 461-79; WO94/13804; Holliger P et al, (1993) Proc. Natl. Acad. Sci. USA, 90: 6444-48) and (ix) scFv genetically fused to the same or a different antibody (Coloma MJ & Morrison SL (1997) Nature Biotechnology, 15(2): 159-163).

The term "monoclonal antibody" as used herein, refers to antibodies that are produced by clone cells all deriving from the same single cell, and that specifically bind the same epitope of the target antigen. When therapeutic antibodies are produced, the generation of monoclonal antibodies is preferred over polyclonal antibodies. In fact, while monoclonal antibodies are produced by cells originating from a single clone and bind all the same epitope, polyclonal antibodies are produced by different immune cells and recognize multiple epitopes of a certain antigen. Monoclonal antibodies assure batch to batch homogeneity, reduced cross-reactivity and high specificity toward the target. Monoclonal antibodies can be expressed, for instance in host cells, using recombinant DNA, giving rise to a recombinant antibody.

The term "recombinant antibody" as used herein, refers to an antibody that has been produced by any process involving the use of recombinant DNA. A recombinant antibody can be engineered in such a way to improve characteristics such as immunogenicity, binding affinity, molecular size, specificity, half-life, and format. Examples of recombinant antibodies include, but are not limited to engineered antibodies, chimeric antibodies, CDRs grafted antibodies (such as humanized antibodies), fully human antibodies, antibody fragments, Fc-engineered antibodies, multispecific antibody (such as bispecific, trispecific, tetraspecific antibody), monomeric and multimeric antibodies (such as homo-dimeric and hetero-dimeric antibodies).

As used herein the terms "therapeutic antibody" or "therapeutic recombinant antibody" are interchangeable and they indicate the use of an antibody or antibody fragment thereof, such as a recombinant antibody or antibody fragment thereof, as therapy. Such antibody might be used for instance for the treatment or prevention of a disease. The term "treatment" or "treating" in the present invention is meant to include therapeutic treatment, as well as prophylactic, or suppressive measures for a disease or disorder. Thus, for example, successful administration of an antibody prior to onset of the disease results in treatment of the disease. As another example, successful administration of an antibody after clinical manifestation of the disease to combat the symptoms of the disease comprises treatment of the disease. "Treatment" and "treating" also encompasses administration of an antibody after the appearance of the disease in order to eradicate the disease. Successful administration of an antibody after onset and after clinical symptoms have developed, with possible abatement of clinical symptoms and perhaps amelioration of the disease, comprises treatment of the disease. Those, such a subject or a patient, "in need of treatment" include mammals already having the disease or disorder, as well as those prone to having the disease or disorder, including those in which the disease or disorder is to be prevented. As used herein, the term "subject" includes any human or nonhuman animal. The term "nonhuman animal" includes all vertebrates, e.g., mammals and non-mammals, such as nonhuman primates, sheep, dogs, cats, horses, cows, chickens, amphibians, reptiles, etc. Preferably the subject is human. A "patient" for the purposes of the present invention includes both humans and other animals, preferably mammals and most preferably humans. Thus therapeutic recombinant antibody have both human therapy and veterinary applications. therapeutic recombinant antibody can be administered via one or more routes of administration using one or more of a variety of methods known in the art. As will be appreciated by the skilled artisan, the route and/or mode of administration will vary depending upon the desired results. Preferred routes of administration include intravenous, intramuscular, intradermal, intraperitoneal, subcutaneous, spinal or other parenteral routes of administration, for example by injection or infusion. More preferred routes of administration are intravenous or subcutaneous. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. Alternatively, an antibody of the invention can be administered via a non- parenteral route, such as a topical, epidermal or mucosal route of administration, for example, intranasally, orally, vaginally, rectally, sublingually or topically

The term "cell transfection" refers to the introduction of foreign genetic material into a cell, such as an eukaryotic cells. When the protein codified by the artificially introduced nucleic acid is expressed by the cells, it provides the genetically modified cells with properties different than the respective wild type form. The introduced nucleic acid can be DNA or RNA. Examples of techniques commonly used for introducing exogenous nucleic acid into the host cells include chemical-based methods, where the transfection is mediated by transfection reagents such calcium-phosphate, liposomes, cationic polymers or dendrimers; physical-based method such as electroporation and microinjection; and virus-based methods where virus infection mediates gene delivery. Using these techniques, transient or stable transfection can be achieved. In the transient transfection the nucleic acid sequence does not integrate into the genome of the host cell, therefore the expression of the protein codified by the exogenous genetic material is limited in time, while stable transfection is achieved when the cells integrate the foreign genetic material in their genome, giving rise to a stable transfected cell line.

A protein, such as an antibody, can be produced by introducing genetic material encoding the protein of interest in host cells. The term "host cells" refers to all the cells in which the protein of interest codified by the artificially introduced genetic material is expressed, including those cells in which the foreign nucleic acid is directly introduced and their progeny. In the host cells it can be introduced an expression vectors (constructs), such as plasmids and the like, encoding the protein of interest e.g., via transformation, transfection, infection, or injection. Such expression vectors normally contain the necessary elements for the transcription and translation of the sequence encoding the biomolecule of interest. Methods which are well known to and practiced by those skilled in the art can be used to construct expression vectors containing sequences encoding the protein of interest, as well as the appropriate transcriptional and translational control elements. These methods include in vitro recombinant DNA techniques, synthetic techniques, and in vivo genetic recombination.

Cell lines suitable for the expression of the antibody include and are not limited to bacteria, mammalian, insect, plant and yeast cells. Cell lines often used for the expression and production of therapeutic antibodies are mammalian cells lines such as Chinese hamster ovary (CHO) cells, NSO mouse myeloma cells, human cervical carcinoma (HeLa) cells and human embryonic kidney (HEK) cells. Host cells are cultured in conditions that aid their growth and the expression of the antibody. Optimal culturing conditions are obtained by the control and adjustment of several parameters including: the formulation of the cell culture medium, the bioreactor operating parameters, the nutrient supply modality and the culturing time period. The formulation of the culturing medium has to be optimized to favorite cell vitality and reproduction; examples of constituents of the cell culture medium include but are not limited to essential amino acids, salts, glucose, growth factors and antibiotics. Important bioreactor operating parameters are: temperature, pH, agitation speed, oxygenation and carbon dioxide levels. Nutrients can be supplied in different ways: in the batch mode culture all the necessary nutrients are present in the initial base medium and are used till exhausted while wastes accumulate; in the fed-batch culture additional feed medium is supplied to prevent nutrient depletion and prolong the culture; differently, in the perfusion modality, cells in culture are continuously supplemented with fresh medium containing nutrients that flows in the bioreactor removing cell wastes. The culturing period is important as it needs to be long enough to let the cells produce a consistent amount of product but it cannot be too long to impair cell viability.

In a particular embodiment of the present invention the cultured cells are mammalian cells, more in particular, they are CHO cells. In an even more specific embodiment, the cells cultured in the present invention are CHO-S cells or HD-BIOP3 cells.

The terms "cell culture" and "culture" as used herein are interchangeable and refer to the growth and/or propagation and/or maintenance of cells in controlled artificial conditions, and they indicate a cell culture which comprises a cell culture medium and cell culture material comprising cells, cell debris, for instance generated upon cell death, colloidal particles, such as DNA, RNA and host cell proteins (HCP), and (bio)molecules secreted by the cultured cells, such as the protein of interest. The cells of a cell culture can be cultured in suspension or attached to a solid substrate, in containers comprising a cell culture medium. For example a cell culture can be grown in tubes, spin tubes, flasks, bags, roller bottles, bioreactors.

Host cell proteins (HCPs) are proteins produced or encoded by the host organisms used to produce recombinant therapeutic proteins. The risks associated with HCPs are primarily immunogenicity. For this reason, HCP should be minimized at the end of a purification process. According to the Food and Drug administration (FDA) "Whenever possible, contaminants introduced by the recovery and purification process should be below detectable levels using a highly sensitive analytical method"; the European Medicines Agency (EMA) guideline CPMP/BWP/382/97 states that for HCP, whatever the product and production system, residual HCP have to be tested for on a routine basis". It is generally accepted that a sensitive, validated method is required to monitor residual HCPs in accordance with ICH guidelines (CPMP/ICH/365/96). The allowed amount of residual HCPs in final bulk material is determined on a case-by-case basis, commonly in the 1 to 100 ng/mg range (Host-Cell Protein Measurement and Control, Wang et al, BioPharm International, Jun 0.1, 2015 Volume 28, Issue 6, pg 32-38).

Often host cells are cultured in bioreactors, under conditions that aid their growth and the expression of the protein of interest. The term "bioreactor," as used herein, refers to any manufactured or engineered device or system that supports a biologically active environment. Optimal culturing conditions are obtained by the control and adjustment of several parameters including: the formulation of the cell culture medium, the bioreactor operating parameters, the nutrient supply modality and the culturing time period. The formulation of the culturing medium has to be optimized to favorite cell vitality and reproduction; examples of constituents of the cell culture medium include but are not limited to essential amino acids, salts, glucose, growth factors and antibiotics. Important bioreactor operating parameters include: initial cell seeding density, temperature, pH, agitation speed, oxygenation and carbon dioxide levels. Nutrients can be supplied in different ways: in the batch mode culture all the necessary nutrients are present in the initial base medium and are used till exhausted while wastes accumulate; in the fed-batch culture additional feed medium is supplied to prevent nutrient depletion and prolong the culture; differently, in the perfusion modality, cells in culture are continuously supplemented with fresh medium containing nutrients that flows in the bioreactor removing cell wastes. The culturing period is important as it needs to be long enough to let the cells produce a consistent amount of product but it cannot be too long to impair their viability. Commonly used bioreactors are typically cylindrical, ranging in size from liters to cubic meters, and are often made of stainless steel. In the embodiments described herein, a bioreactor is made of plastic or of stainless steel. It is contemplated that the total volume of a bioreactor may be any volume ranging from 100 ml to up to 20.000 Liters or more, depending on a particular process.

The terms "cell culture medium," and "culture medium" and "medium" are used interchangeably herein and they refer to a nutrient solution used for growing cells, such as animal cells, e.g., mammalian cells. Such a nutrient solution generally includes various factors necessary for cell attachment, growth, and maintenance of the cellular environment. For example, a typical nutrient solution may include a basal media formulation, various supplements depending on the cell type and, occasionally, antibiotics.

The terms "clarify", "clarification", "clarification step", "clarification process" as used herein are interchangeable and generally refer to one or more steps that aid the removal of material from the cell culture, such as removal of cells, cell debris and colloidal particles, to obtained clarified cell culture. The obtained clarified cell culture, also called herein "clarified cell harvest" or "clarified cell harvest material" interchangeably normally then undergoes downstream processing steps to purify the protein of interest contained in the clarified harvest. Clarification can be carried out via one or more steps of centrifugations and/or filtrations.

The term "viral clearance" refers to any treatment that effectively remove and/or inactivate viruses which could contaminate the material of interest. When applied in the purification process of a therapeutic antibody, virus clearance refers to any method that lead to viral inactivation or viral removal from an antibody-containing material.

The term "viral inactivation" refers to any treatment that makes a virus unable to infect biological samples or to replicate. Viral inactivation can be achieved by different techniques such as the incubation of the biological sample with solvents and detergents (S/D), which causes viral inactivation trough the solubilization of the viral envelope; the incubation in low or high pH, which leads to the denaturation of the viral proteins; the pasteurization treatment, in which viral protein denaturation is achieved by high temperatures. Downstream processes normally have two orthogonal steps for viral clearance, basically a physical and a chemical one (European Medicines Agency guidelines). Generally a chemical inactivation is done on PA eluates as enveloped viruses (retroviruses, herpesviruses, parvoviruses, etc) are sensitive to this treatment. It can be achieved by addition of a solvent / detergent mixture or by decreasing / increasing the pH to extreme values.

The term "chromatography" refers to the operation of separating compounds of a mixture based on their capability to interact with a stationary phase of a chromatography column, from which they can be retained or eluted. Chromatography techniques are known in the art, for instance ion exchange chromatography separates ions and polar molecules based on their affinity to the ion exchange, example of ion exchange chromatography techniques are cation exchange chromatography and cation exchange chromatography. Generally in the purification process of a protein, such as an antibody, they aid the removal of impurities such as host DNA, endotoxins, host cell proteins and eventually viruses.

In the present invention the yield of a purification step or of two or more subsequent purification steps is calculated as the percentage of the ratio between the amount of the protein of interest obtained after said step(s) and the amount present in a protein solution before said step.

According to one aspect of the present invention the disclosed method for preparing a purified therapeutic recombinant antibody solution from a clarified cell harvest, comprises the steps of (i) subjecting the clarified cell harvest to (i) an affinity chromatography step, (ii) a viral inactivation step, (iii) at least two steps of ion exchange chromatography. In a more particular aspect of the present invention, affinity chromatography is selected from the group comprising Protein A chromatography, Protein G chromatography and Protein L chromatography. In a preferred aspect of the present invention affinity chromatography is Protein A chromatography. In particular embodiment of the present invention, protein A chromatography is carried out by first equilibrating the column with PBS at pH 7.4; secondly by loading the the clarified cell harvest into the column at a loading factor ≥ 5g/Lᵣₑₛᵢₙ and ≤ 34g/Lᵣₑₛᵢₙ; next by washing the column with PBS pH 7.4; and finally by eluating the product with 0.1 M Glycine pH 3.5.

According to one aspect of the present invention, the viral inactivation step is performed with a method selected from the group comprising: low pH treatment; high treatment; treatment with solvents and detergents. Preferably the viral inactivation is performed with a pH treatment; buffer solutions useful for a pH treatment include alkaline solution including, but not limited to acetate, citrate, Tris, Histidine, L-Arginine, phosphate, NaOH. Non limiting examples of low pH treatment include pH lower than about 6.5 and higher than about 1. Non limiting examples of high pH treatment include pH greater than about 7.5 and less than about 14. In one embodiment of the present invention, viral inactivation is carried out at a pH or about 3.5 with 3.7%HCl, for an incubation time comprised between about 60 min and about 90 min. According to one aspect of the present invention, viral inactivation is performed on the protein A chromatography eluate and the resulting viral inactivated solution undergoes the first of the at least two steps of ion exchange chromatography. According to a further aspect of the present invention, the viral inactivated solution is first neutralized undergoing a ion exchange chromatography step. Preferably the viral inactivated solution is neutralized to reach the same pH of the elution buffer used in the following ion exchange chromatography, and with the same buffer.

In one aspect of the present invention, after the first of the at least two steps of ion exchange chromatography the pH of the ion exchange chromatography eluate is adjusted so as to be the same of the elution buffer of the following step of ion exchange chromatography, using the same buffer.

In one embodiment of the present invention, the at least two steps of ion exchange chromatography comprise a first step of cation exchange chromatography and a second step of anion exchange chromatography. In another embodiment of the present invention, the at least two steps of ion exchange chromatography comprise a first step of anion exchange chromatography and a second step of cation exchange chromatography.

In a particular aspect, cation exchange chromatography (CEX) is carried out in bind-eluate mode wherein the antibody is eluted with an elution buffer selected from the group comprising Citrate, Acetate and Phosphate. In a more particular aspect, CEX elution is carried out with an elution buffer comprising between about 10 mM and about 50 mM of Citrate, more specifically between about 20 mM and about 30 mM of Citrate, and at least 30 mM of NaCl, more specifically between about 50 mM and about 120 mM of NaCl at pH between about 5 and about 6. In a preferred embodiment, CEX elution is carried out with an elution buffer comprising about 25 mM of Citrate, and between about 100 mM or about 60 mM of NaCl at pH of about 5.2, or about 5.5. In a particular embodiment of the present invention, CEX is carried out by first equilibrating the column with 25 mM Citrate at pH 5.5; secondly by loading the sample into the column at a loading factor of about 45g/Lᵣₑₛᵢₙ; next by washing the column with 25 mM Citrate and 20 mM or 40 mM NaCl at pH 5.5; and finally by eluting the product with 25 mM of Citrate, and between about 100 mM or about 60 mM of NaCl at pH 5.5.

In another particular aspect, anion exchange chromatography is carried out by membrane absorption (MA) in flow-through mode using a buffer selected from the group comprising Tris and Histidine. In a more particular aspect, MA is carried out with a buffer comprising between about 20 mM and 60 mM of Histidine or Tris buffer at a pH between about 6.5 and about 8. In a preferred embodiment, MA is carried out with a buffer comprising about 50 mM of Histidine, at pH selected from the group comprising 6.5, 7, 7.2, 7.4. In a particular embodiment of the present invention, MA is carried out using about 50 mM Histidine at a pH between about 7 and about 7.5 at a flow rate (FR) comprised between about 2 MV/min and about 30 MV/min, and with a loading factor (LF) comprised between about 1g/mL and about 15 g/mL. In a more particular embodiment, MA is carried out using about 50 mM Histidine at a pH selected from the group comprising about 7, about 7.2 and about 7.4, at a flow rate selected from the group comprising about 5 MV/min and about 25 MV/min, with a loading factor selected from the group comprising about 2.5 g/mL, about 5 g/mL, about 7.5 g/mL and about 10 g/mL. In a preferred embodiment, MA is carried out using about 50 mM Histidine at a pH 7.2 at a flow rate of 25 MV/min, with a loading factor of 10 g/mL. Specifically, the MA steps of the most preferred embodiment is carried out by using the following parameters:
- Venting (50 MV): 50 Mm Histidine, pH 7.2 FR 25 MV/min:
- CIP: 0.5 M NaOH, 2 NaCl, FR 10 MV/min during 5 min + 60 min static soack
- Strip (150MV): 2 M NaCl, FR 10 MV/min
- Equilibration (50 MV): 50 M Histidine pH 7.2, FR 25 MV/min
- Load: ∼10 M Histidine pH 7.2, FR 25 MV/min, LF 10 g/mL
- Flush (40 MV): 50 mM Histidine pH 7.2, FR 25 MV/min

According to one aspect of the present invention, the therapeutic recombinant antibody solution obtained by the method of the present invention comprises less than about 40 ppm of host cell proteins. Preferably the therapeutic recombinant antibody solution obtained by the method of the present invention comprises less than about 30 ppm; more preferably less than about 20 ppm. In preferred embodiments of the present invention the therapeutic recombinant antibody solution obtained by the method of the present invention comprises less than about 15 ppm; in more preferred embodiments of the present invention the therapeutic recombinant antibody solution obtained by the method of the present invention comprises less than about 10 ppm; in an even more preferred embodiments of the present invention the therapeutic recombinant antibody solution obtained by the method of the present invention comprises less than about 5 ppm.

According to another aspect of the present invention wherein the yield of the at least two ion exchange chromatography steps said method is higher than about 70%, preferably the yield of said method is higher than about 80%.

According to a further aspect of the present invention, the therapeutic recombinant antibody solution obtained by the method disclosed herein comprises less than about 2 pg/mg DNA, preferably less than about 1 pg/mg DNA, more preferably less than about 0.8 pg/mg DNA.

In one embodiment of the present invention, the therapeutic recombinant antibody solution obtained by the method of the present invention comprises less than about 5 ppm of HCP, less than 0.8 pg/mg of DNA and it has a yield higher than 80%.

In one aspect of the present invention wherein the protein titer of the clarified cell harvest is comprised between about 0.5 g/L and about 10 g/L.

Also disclosed by the present invention is a process for preparing a purified therapeutic recombinant antibody solution from a clarified cell harvest, comprising the steps of:
(i) Protein A chromatography of a clarified cell harvested antibody material;
(ii) Incubation of the resulting PA eluate at pH 3.5;
(iii) Neutralization of the resulting viral inactivated solution to pH7.2, followed by 0.2 µm filtration;
(iv) Anion exchange chromatography of the neutralized viral inactivated solution according to claim 11, followed by 0.2 µm filtration;
(v) Cation exchange chromatography of the anion exchange chromatography eluate according to claim12, followed by 0.2 µm filtration.

The current invention also relates to a process for the production of a bulk drug substance comprising the steps of:
(i) Protein A chromatography of a clarified cell harvested antibody material;
(ii) Incubation of the resulting PA eluate at pH 3.5;
(iii) Neutralization of the resulting viral inactivated solution to pH7.2, followed by 0.2 µm filtration;
(iv) Anion exchange chromatography of the neutralized viral inactivated solution according to claim 11, followed by 0.2 µm filtration;
(v) Cation exchange chromatography of the anion exchange chromatography eluate according to claim12, followed by 0.2 µm filtration;
(vi) Virus nanofiltration;
(vii) Concentration of the product by ultrafiltration and continuous diafiltration into preformulation buffer, followed by 0.2 um filtration;
(viii) Excipient addition;
(ix) 0.2 µm filtration.

The present invention also relates to the purified therapeutic recombinant antibody solution or the bulk drug substance obtained by said processes.
Figure 1. Chromatogram of CEX steps in tested conditions A, B and C with PP1
Figure 2. Chromatogram of CEX steps in tested conditions A and B with PP1
Figure 3. Chromatogram of MA steps in tested condition A with PP1
Figure 4. Chromatogram of MA steps in tested condition C with PP1
Figure 5. Chromatogram of MA steps for all conditions tested with PP2
Figure 6. Chromatogram of CEX steps for all conditions tested with PP2
Figure 7. Chromatogram of the Ab1 MA step of the preliminary studies. The different steps are identified on the x axis. The UV, conductivity, pre-column pressure and delta column pressure curves are respectively represented in red, blue green and purple.
Figure 8. Graphical representation of the 27 DoE MA runs analytical results
Figure 9. Distribution and quantile box of the DoE responses
Figure 10: Prediction profiler of the model
Figure 11. Comparison PP2 applied on Ab1 and Ab2
Figure 12: Comparison RPP and PP2

### EXAMPLES

### Example 1: Development of the purification processes PP1 and PP2

In order to optimize the purification process of the monoclonal antibody Ab1, two purification processes have been developed. The first purification process (PP1) is characterized by the steps of:
- Affinity chromatography;
- Virus inactivation;
- Cation exchange chromatography;
- pH adjustment;
- Anion exchange chromatograph by membrane absorption in flow-through mode;
- Virus filtration;
- Ultrafiltration/Diafiltration.

The second purification process (PP2) is characterized by the steps of
- Affinity chromatography;
- Virus inactivation;
- Anion exchange chromatograph by membrane absorption in flow-through mode;
- pH adjustment;
- Cation exchange chromatography;
- Virus filtration;
- Ultrafiltration/Diafiltration.

The two processes PP1 and PP2 were compared to a reference purifications process (RPP) characterized by the steps of:
- Affinity chromatography;
- Virus inactivation;
- Cation exchange chromatography;
- Ultrafiltration/Diafiltration (UF/DF1);
- Anion exchange chromatograph by membrane absorption in flow-through mode;
- Virus filtration;
- Ultrafiltration/Diafiltration (UF/DF2).

The focus of the new purification processes (PP1 and PP2) development was directed to the two ion exchange chromatography steps, namely cation exchange chromatography (CEX) and anion exchange chromatograph (AEX) by membrane absorption (MA). The conditions and the order to carry out these steps where improved so as to obtain a surprisingly high HCP reduction and high purity.

### Material and methods

### Biological material

Ab1 was produced in S-CHO cells under controlled culturing conditions.

### Chemicals and buffers

All chemicals used were pharmacopoeia grade and purchased from well-established companies. The water used to manufacture the buffers was ISO 3696 Type II.

### Process intermediates management

All process intermediates were 0.2 µm filtered before being loaded and after the step so as to avoid resin/membrane clogging and biological contamination of intermediates. Generally, product intermediates were stored at 5°C ± 3°C in a cold room. Retains and samples for analysis were frozen at - 20°C and thawed before use. Intermediates were brought to room temperature (20 - 25°C) before a purification step.

### Protein A chromatography

The purpose of the capture step is to remove most of the components of the cell-culture supernatant while maintaining a high recovery. The chromatography method of choice for antibodies is affinity chromatography using a protein A resin. The protein A step was performed with MabSelect Sure resin (GE Healthcare) and according to the following conditions.. First the column was equilibrated with PBS at pH 7.4The harvest clarified with dead-end depth filtration was loaded into the column at a loading factor ≥ 5g/Lᵣₑₛᵢₙ and ≤ 34g/Lᵣₑₛᵢₙ. The column was washed using PBS pH 7.4 and the product was eluted with 0.1 M Glycine pH 3.5.The PA eluate containing Ab1 was produced during a 5000 L clinical run with a CHO-S.

### Viral inactivation

Viral inactivation of PA eluates were done at low pH. First, sufficient PA eluate volume was brought to pH 3.5 with HCl 3.7% for an incubation ≥ 60 minutes and ≤ 90 minutes. Following the incubation, a neutralization to the desired pH with 250 mM Histidine pH 12.0 or 0.5 M Citrate pH 12.0 was done.

### Analytical methods

Product quality was monitored after purification steps with the methods are presented in the Table 1.

**Table 1: Product quality analysis**

| Analysis | Characteristics | Purpose |
|---|---|---|
| HPLC-SE | Purity | Determination of monoclonal antibodies purity in terms of aggregates and monomer |
| cGE reduced and non-reduced | Identity/purity | Determination of samples purity in terms of fragments and non-fragmented IgG |
| cIEF by ICE3 | Charge variants / pl | Determination of the IEF profile of monoclonal antibodies in purified samples by capillary electrophoresis, to be able to distinguish main product from its charged variants |

The IgG monomer purities were determined by High Pressure Liquid Chromatography coupled Size Exclusion Chromatography (HPLC-SE) that allows to separate IgG aggregates from monomers and fragments. The non-fragmented IgG were detected by Capillary Gel Electrophoresis reduced (cGE-R) and non-reduced (cGE-NR). The reduced one just put in evidence the ratio of heavy chain and light chain of an intermediate whereas the non-reduced one permits to identify the whole IgG molecule from fragmented ones. Samples were also evaluated by Capillary Isoelectric Focusing (clEF). With this method antibodies migrate on a gel according to their Isoelectric Point (pi). The migration stops at the gel zone pH corresponding to the molecule pl. This way acidic and basic variant percentages of each antibody can be determined.

### RPP

In the RPP, PA chromatography was performed as previously described. Next, viral inactivation was performed by incubating the PA eluted at low pH (pH 3.5), by acidification with 3.7% HCl during ≥ 60 minutes and ≤ 90 minutes. The product was then neutralized at pH 5.2 using 0.5 M Citrate pH12.0. CEX was performed using Fractogel EMD SO3- (M) resin from Merck Millipore. The column was equilibrated with 25mM Citrate pH 5.2, the VI Neutralized product was loaded into the column at a loading factor ≥ 15g/Lᵣₑₛᵢₙ and ≤ 45g/Lᵣₑₛᵢₙ. The column was washed using 25mM Citrate, 60mM NaCl pH 5.2 and the product was eluted with 25mM Citrate, 100mM NaCl pH 5.2. The following step of the process was the ultrafiltration/diafiltration (UFDF), performed using Sius Cassette HyStream 30KDa LP (TangenX/Repligen). The equilibration buffer was composed of 25mM Citrate, 100mM NaCl pH 5.2. The product was diafiltered in 50 mM Histidine pH 6.5 with ≥ 7 diavolumes. The anion exchange membrane adsorber step (MA) was performed with NatriFlo HD-Q membranes (Natrix) using 50mM Histidine pH 6.5. The viral filtration was done with a nanofilter Virosart HF (Sartorius) using 50mM Histidine pH 6.5. The final ultrafiltration / diafiltration (UFDF2) was performed with Centramate Omega T series 30 KDa (Pall) using 50mM Histidine pH 6.5 for equilibration and the product was diafiltered in preformulation buffer 5 mM Histidine pH 6.0 with ≥ 5 diavolumes.

### PP1

In PP1, PA chromatography was performed as previously described. Next, viral inactivation was performed by incubating the PA eluted at low pH (pH 3.5), by acidification with 3.7% HCl during ≥ 60 minutes and ≤ 90 minutes. The product was then neutralized at pH 5.2 or pH 5.5 with 0.5M Citrate pH 12.0. The following CEX is performed using Fractogel EMD SO3- (M) resin (Merck Millipore), testing the conditions in Table 2.

**Table 2. CEX conditions**

| CEX Condition | Equilibration | Wash | Elution |
|---|---|---|---|
| A | 25mM Citrate, pH 5.5 | 25mM Citrate, pH 5.5 | 25mM Citrate, 60mM NaCl pH 5.5 |
| B | 25mM Citrate, pH 5.5 | 25mM Citrate, pH 5.5 | 25mM Citrate, 80mM NaCl pH 5.5 |
| C | 25mM Citrate pH 5.2 | 25mM Citrate, 60 mM NaCl pH 5.2 | 25mM Citrate, 100mM NaCl pH 5.2 |

For CEX conditions A and C, which led to highest product quality after chromatography (see the following results), the conditions for pH adjustment and anion exchange membrane adsorber reported in Table 3 have been tested.

**Table 3. pH adjustment and MA conditions**

| CEX condition | pH adjustment | MA | Test Name |
|---|---|---|---|
| A | pH 5.5 to pH 6.5, with 250mM Histidine pH 12.0 | 50mM Histidine pH 6.5 | CEX60-MAHis6.5 |
| | pH 5.5 to pH 7.0, with 250mM Histidine pH 12.0 | 50mM Histidine pH 7.0 | CEX60-MAHis7.0 |
| | pH 5.5 to pH 7.5, with 1M Tris-base | 25mM Tris pH 7.5 | CEX60-MATris7.5 |
| C | pH 5.2 to pH 6.5, with 250mM Histidine pH 12.0 | 50mM Histidine pH 6.5 | CEX100-MAHis6.5 |
| | pH 5.2 to pH 7.0, with 250mM Histidine pH 12.0 | 50mM Histidine pH 7.0 | CEX100-MAHis7.0 |
| | pH 5.2 to pH 7.5, with 1M Tris-base | 25mM Tris pH 7.5 | CEX100-MATris7.5 |

### PP2

In PP2, a PA chromatography was performed as described for RPP. Next, viral inactivation was performed by incubating the PA eluted at low pH (pH 3.5), by acidification with 3.7% HCl during ≥ 60 minutes and ≤ 90 minutes. The product was then neutralized at pH 6.5 or pH 7.0 with 250mM Histidine pH 12.0. Two conditions were tested for the following MA, pH adjustment and CEX steps, as indicated in Table 4.

**Table 4. PP2 tested conditions**

| MA | pH Adjustment | CEX (Elution) | Test Name |
|---|---|---|---|
| 50mM Histidine pH 6.5 | pH 6.5 to pH 5.2, with 1M Citrate pH 3.5 | 25mM Citrate, 100mM NaCl pH 5.2 | MAHis6.5-CEX100 |
| 50mM Histidine pH 7.0 | pH 7.0 to pH 5.2, with 1M Citrate pH 3.5 | 25mM Citrate, 100mM NaCl pH 5.2 | MAHis7.0-CEX100 |

### Results and discussion

First the characteristics of the PA eluate resulting from PA chromatography of the PA load comprising Ab1 where analyzed and the results are given in Table 5.

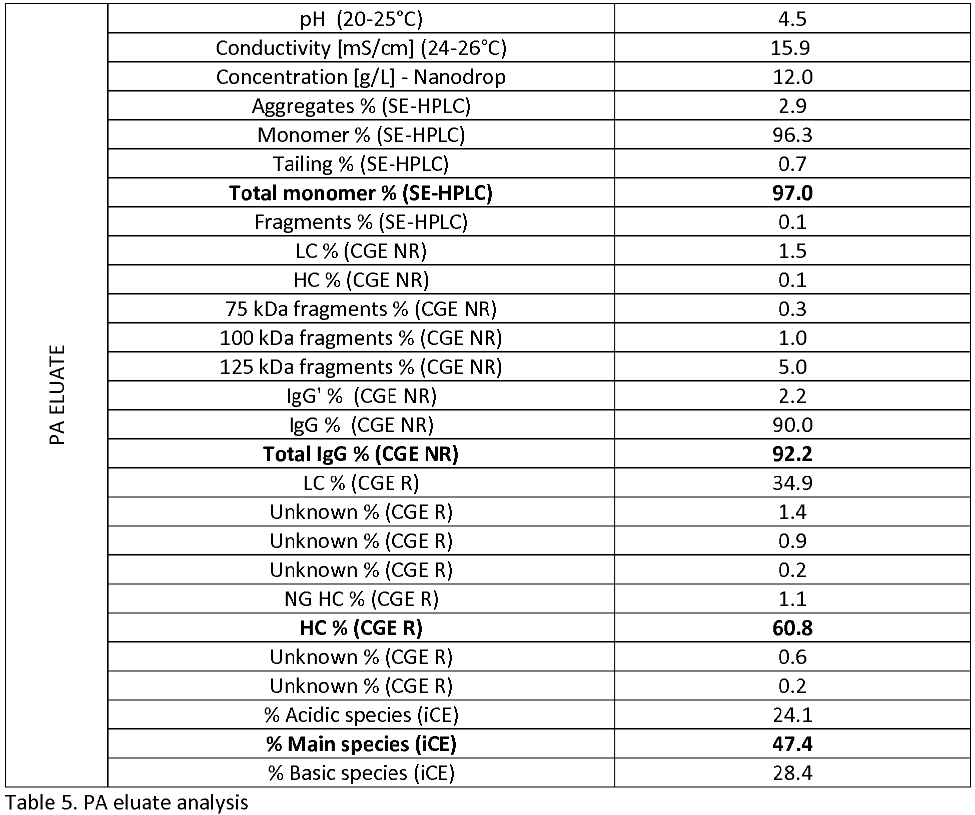

### PP1

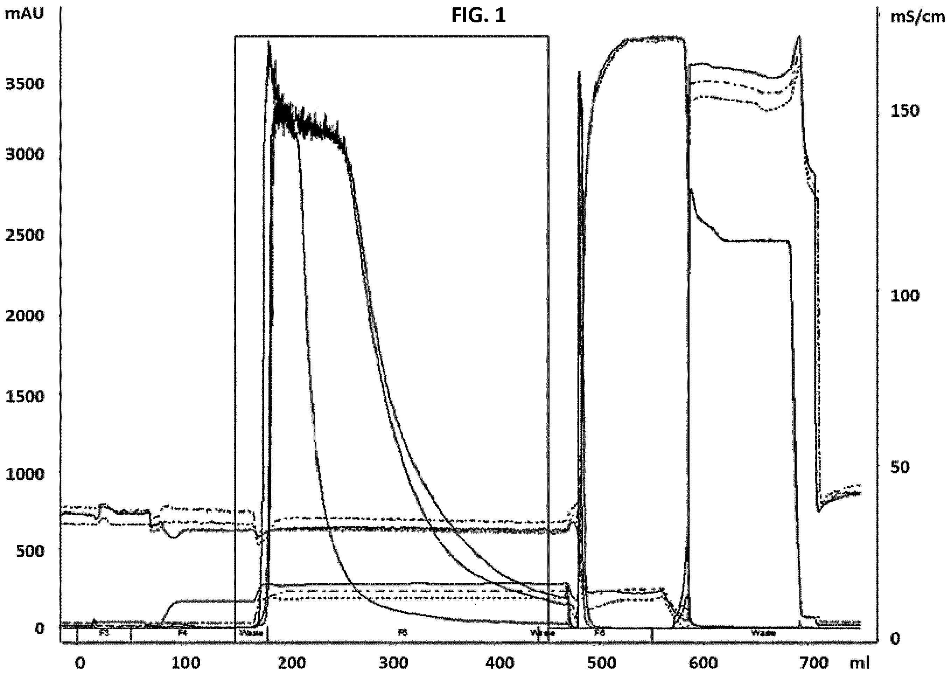

After PA chromatography and VI, the different conditions for performing CEX shown in Table 2 were tested. The results of the product quality in the CEX eluate for each condition are shown in Table 6. Figure 1 shows chromatography profiles.

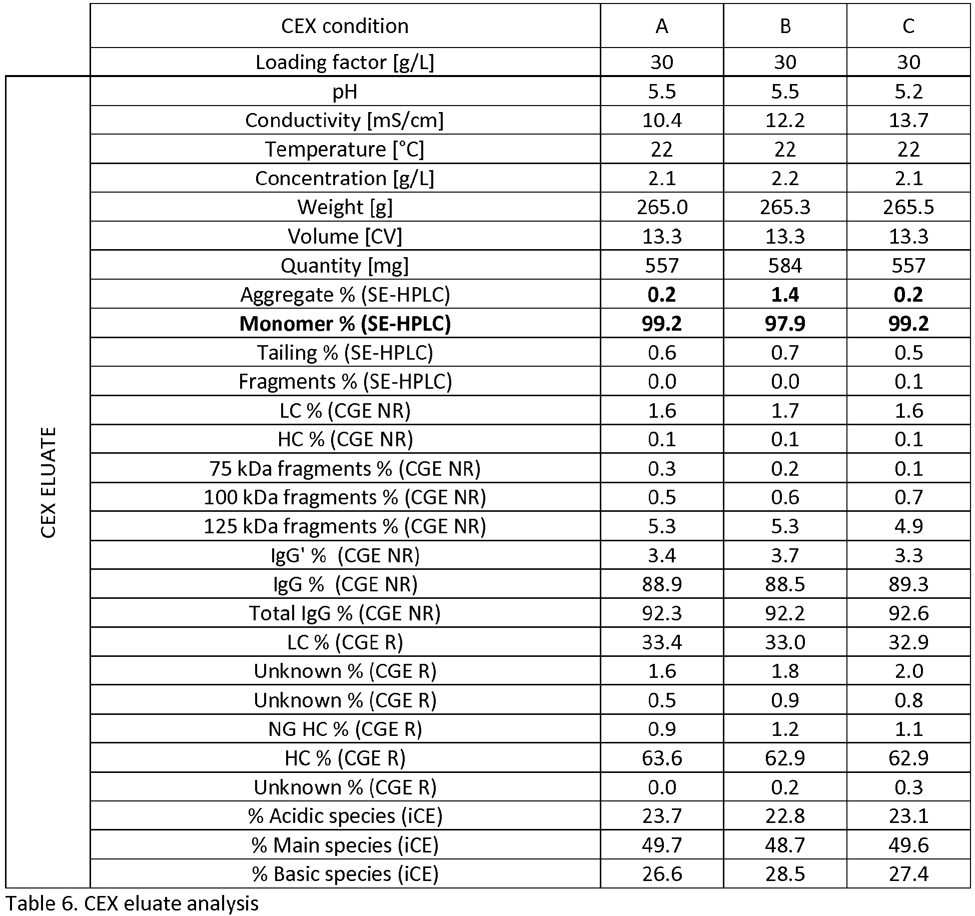

Given that conditions A and C gave comparable chromatographic profiles and better product quality, they were selected for the next steps optimization.

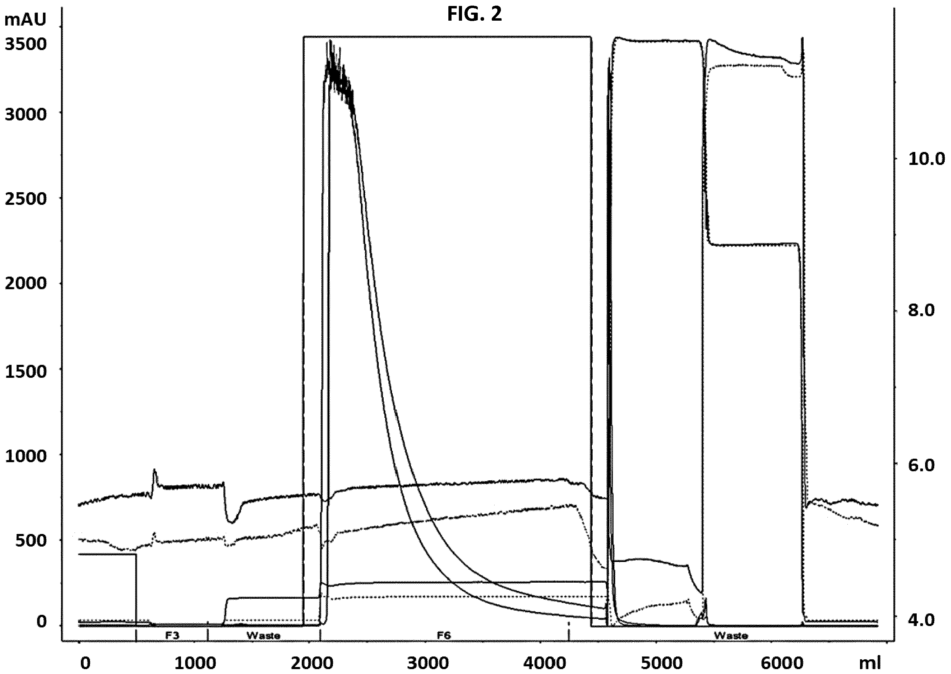

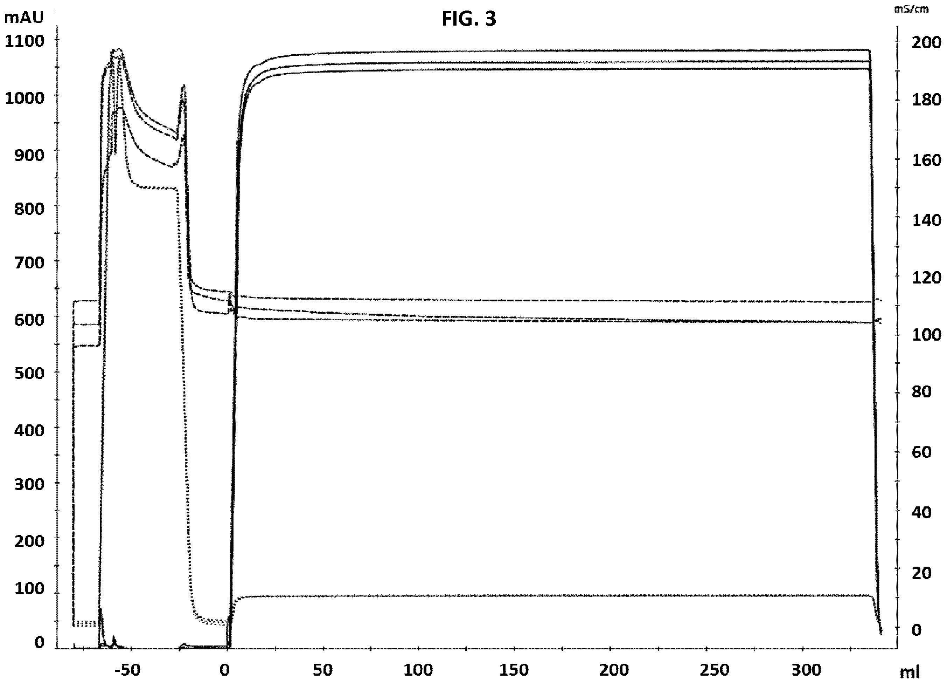

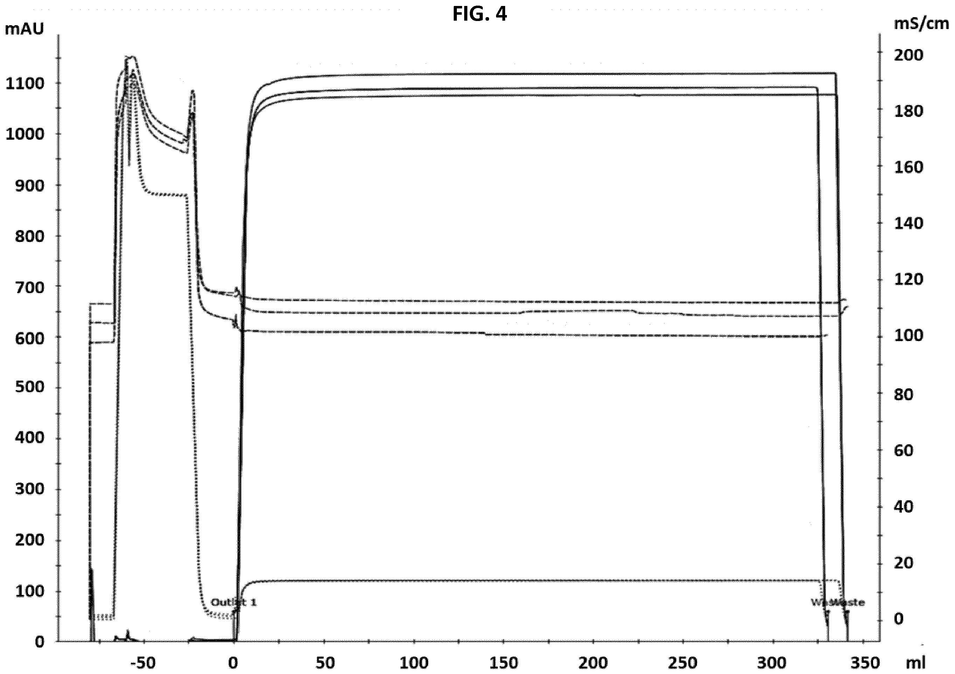

CEX was run at conditions A and C, followed by pH adjustment and MA run according to the conditions of Table 3, product quality was analyzed after the CEX step and after the MA step. The results of the product quality analysis after CEX and MA are given in Table 7 and Table 8, respectively. The corresponding chromatography profiles are given in Figure 2. MA profiles in CEX condition A are shown in Figure 3, MA profile after CEX condition C are shown in Figure 4.

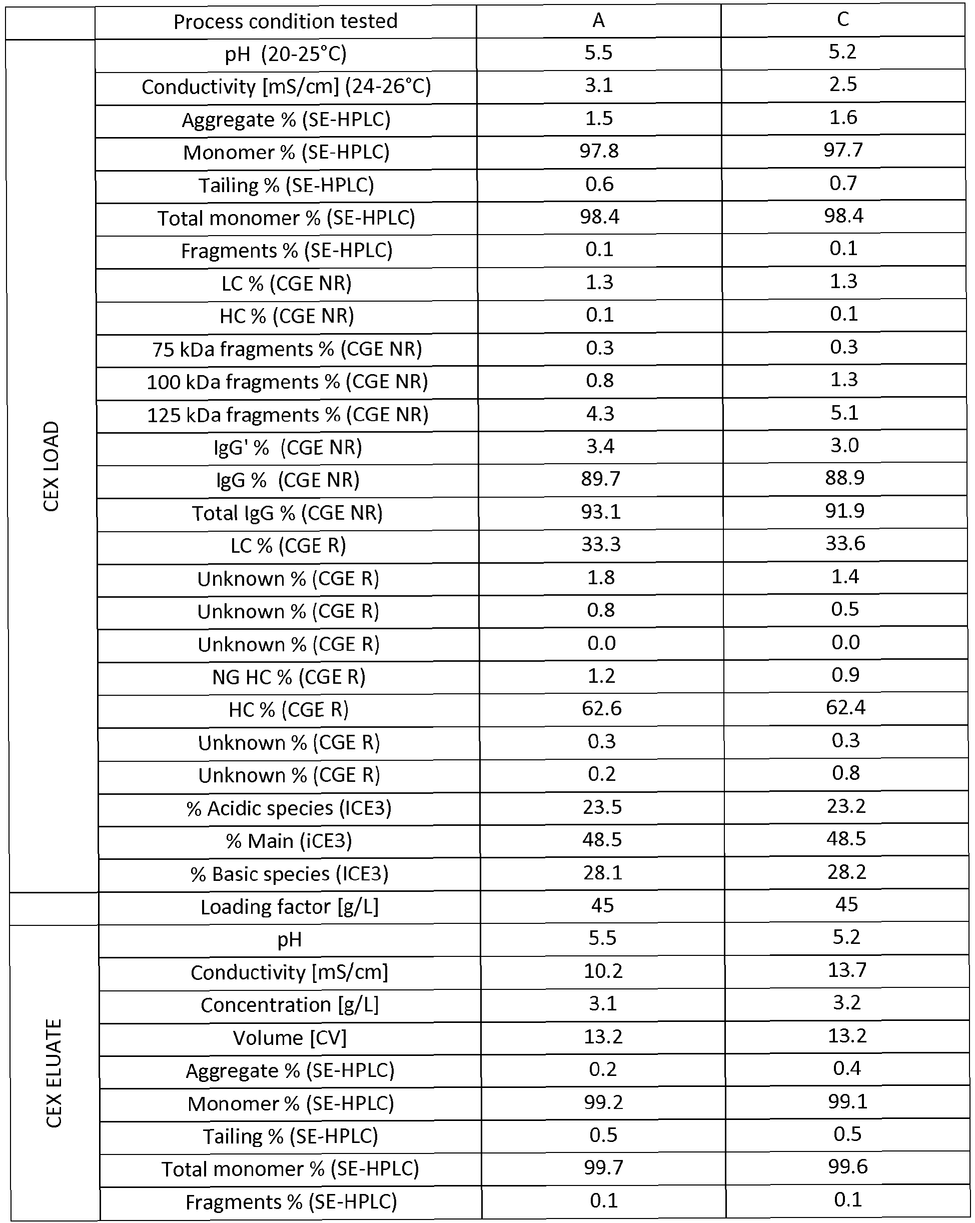

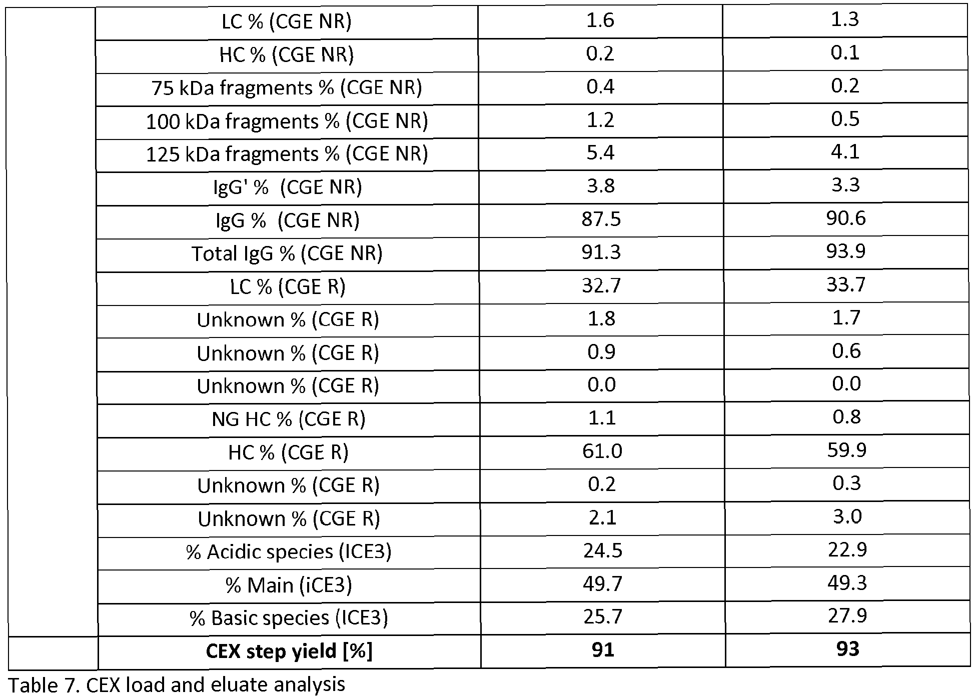

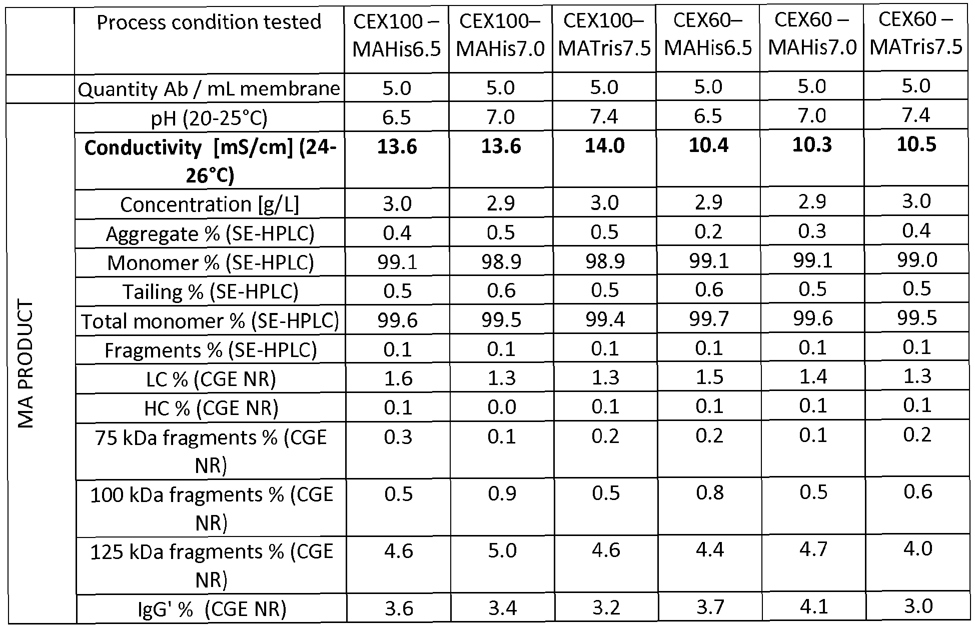

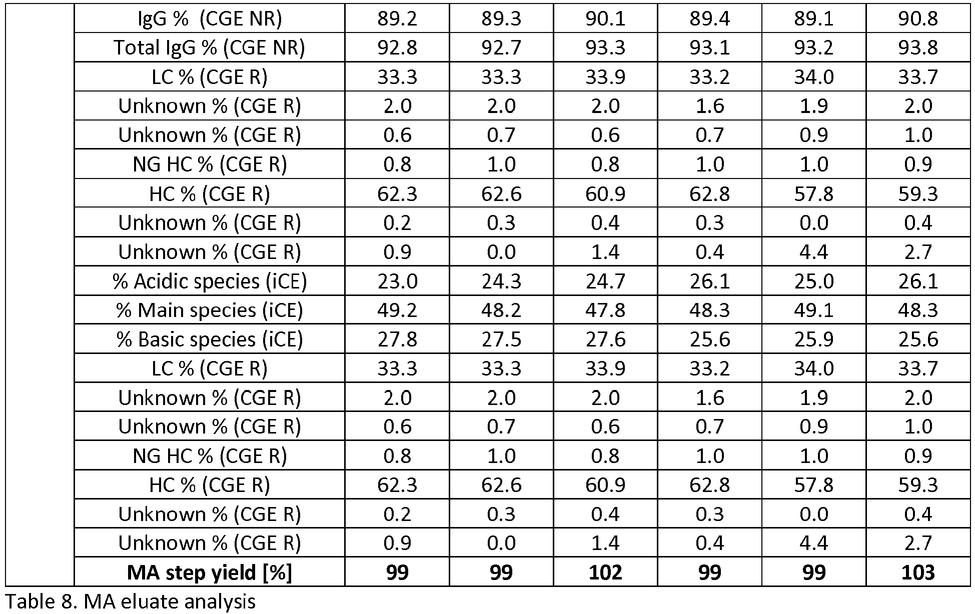

### PP2

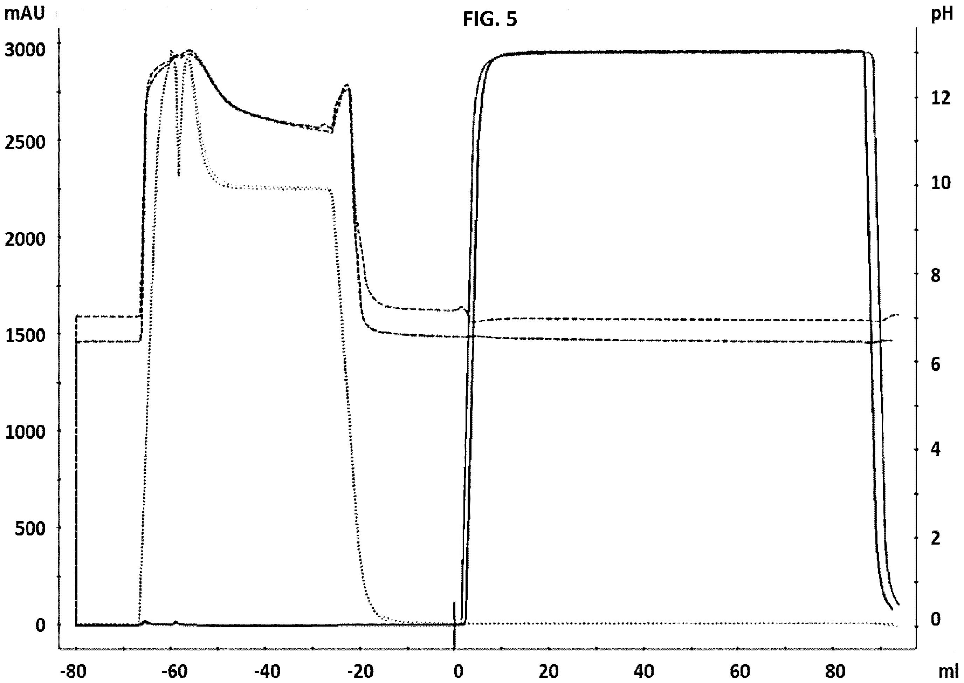

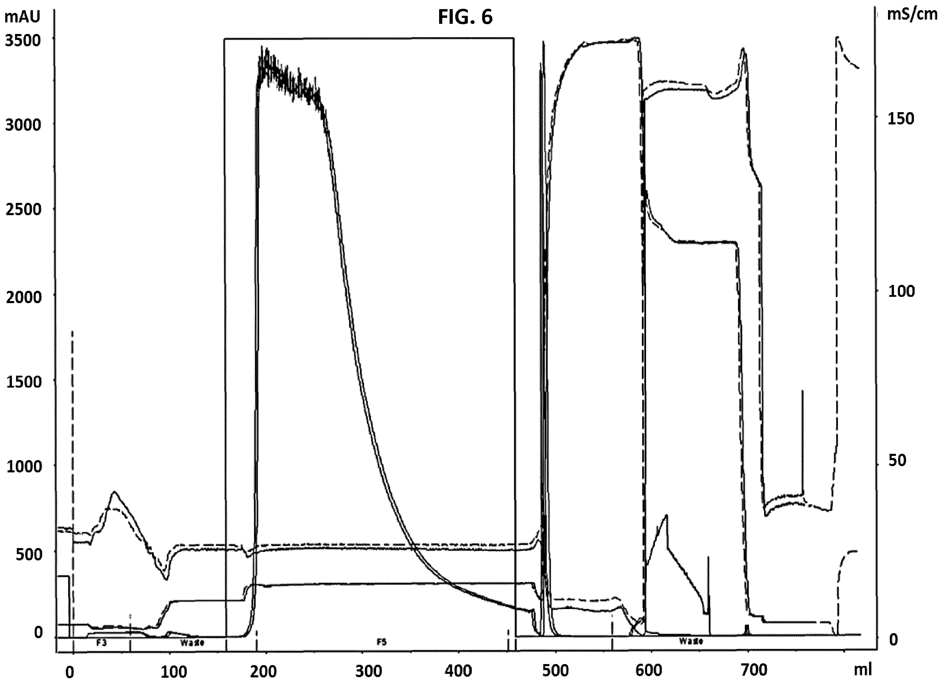

Product quality analysis results obtained for PP2 tested in the conditions of Table 4, are presented in Table 9 for MA load and eluate (MA chromatography profiles are shown in Figure 5) and in Table 10 for CEX eluate (CEX chromatography profiles are shown in Figure 6).

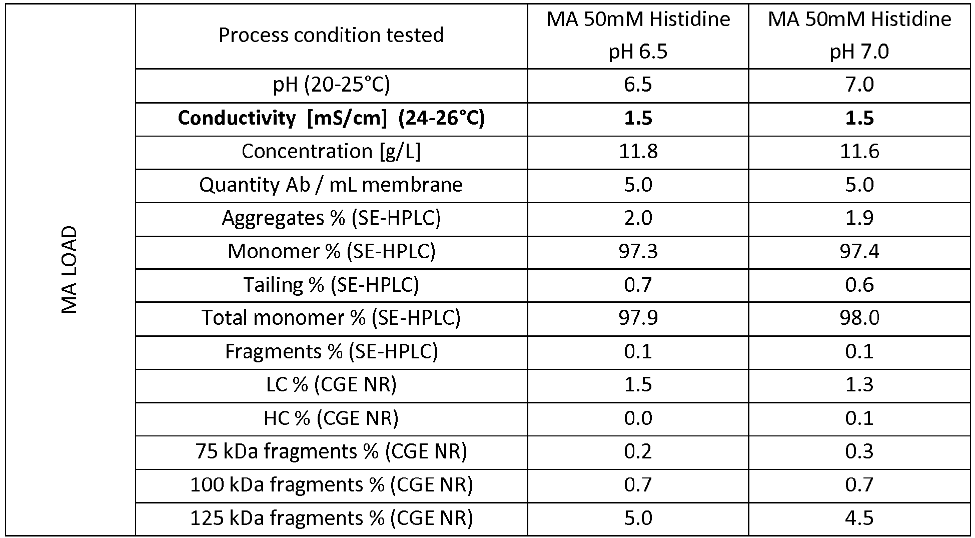

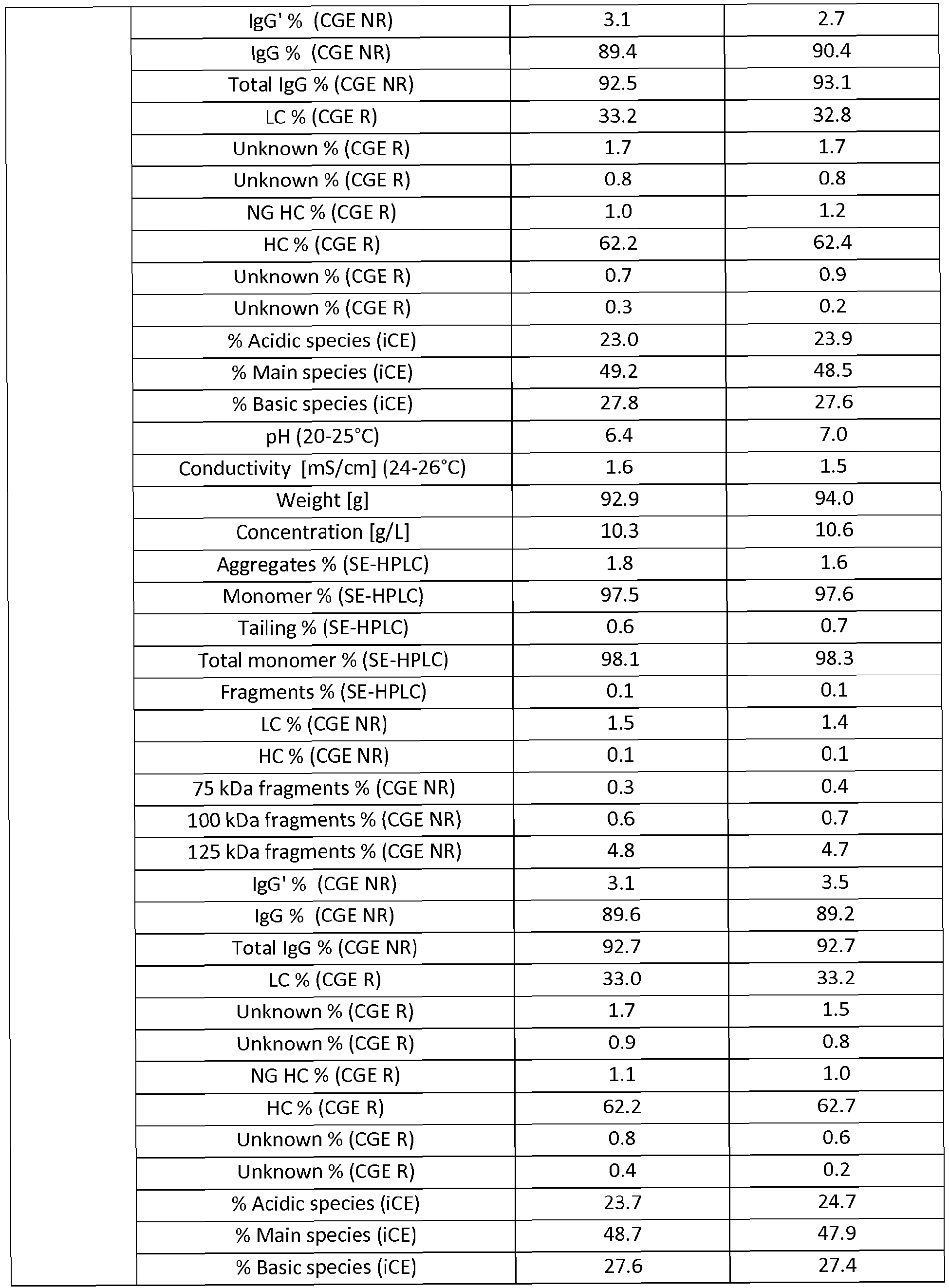

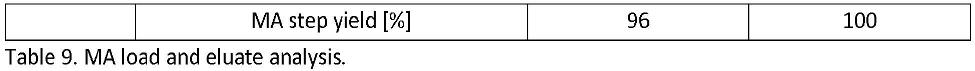

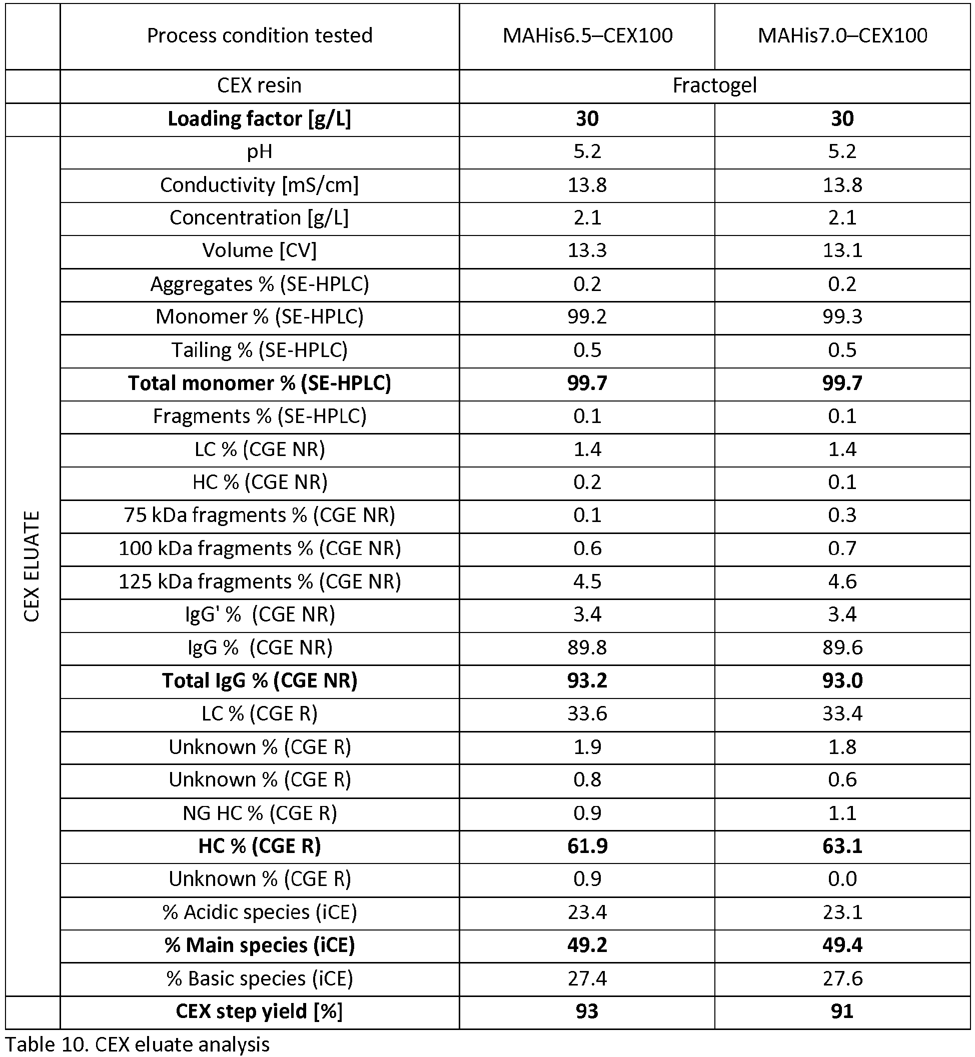

### Comparison PP1, PP2 and RPP

The comparison of the analysis of the product quality for PP1, PP2 and RPP is presented in Table 11.

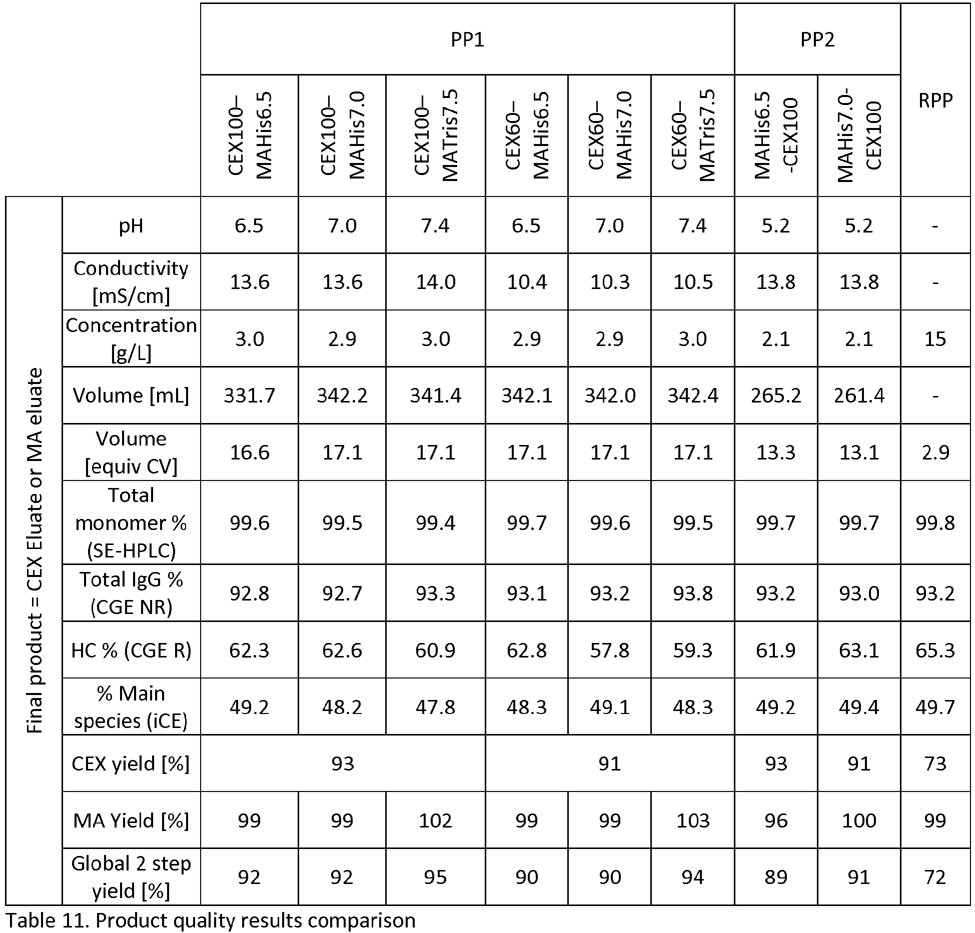

To further investigate the goodness of the tested processes, HCP removal was measured. The results are shown in Table 12.

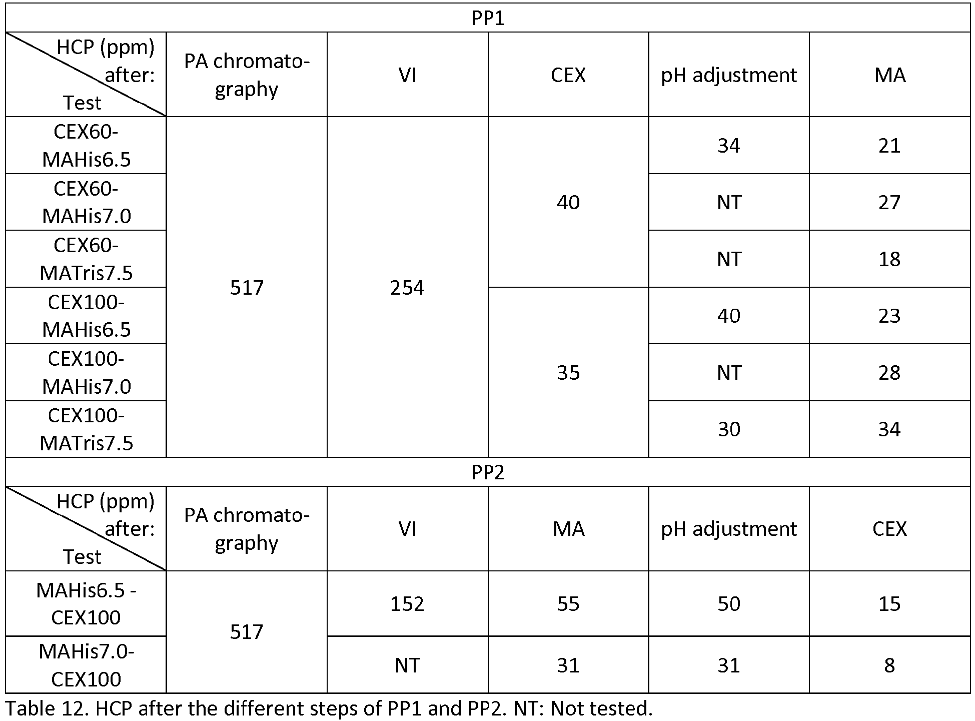

The above results show that the tested conditions for PP1 and PP2 are valuable alternatives to RPP. In fact, the quality of the product as the yields after the two polishing steps (CEX and MA or vice versa) were comparable to RPP. In addition, regarding the level of HCPs after the two polishing steps, PP1 as PP2 showed high HCP removal. Nevertheless, PP2 has demonstrated to have a better capacity to remove HCPs than PP1 with values between 8 and 15 ppm. The HCP removal obtained with PP2 was comparable to RPP which showed levels after polishing steps between 9 and 14 ppm. Consequently, with PP2, MA has demonstrated its capacity to supports to be challenged in term of HCP load, when being the first polishing step. Based on these results, PP2 was the starting point for further investigation, described in example 2 below.

### Example 2: Optimization of the MA step for PP2 - overview

MAHis7.0-CEX100 was used as starting point for the further optimization of the MA step. To this aim the following studies were carried out:
1) Preliminary study. First a preliminary study were done to test the worst conditions for the MA step.
2) Design of experiment (DoE) analysis. DoE analysis allowed to selected and adjusted MA conditions. In particular, DoE is a mathematical and statistical approach to plan experiments, analyze and interpret the results including parameters as reliability of the model, repeatability, distribution, etc. The key advantage of DoE compared to a one-factor-at-the-time (OFAT) approach is to assess the effect of different factors (input) and their interaction on different responses (output). DoE are also useful to minimize the number of experiments if material is expensive and / or in small quantities, whereas OFAT requires much more experiments and material. Among the different existing designs, the full-factorial was chosen for the purpose of this study. As the study was in an optimization context, this design allows to assess the entire experimental space and its extremity. In addition, the available material was sufficient to use this design. At the opposite, fractional design choses some points of the experimental space to test, to reduce the number of runs needed, and is consequently more appropriate in case of screening parameters. The choice of the factors to assess for the MA optimization and their values were determined following the preliminary studies, the literature, the DSP team observations and the supplier recommendations. The pH step values (for equilibration, load, flush) were chosen in the highest limit of the Histidine buffer range while not being too close to the Ab pl (pl Ab1= 8.13). This was done to target the removal of the broadest HCP population at the highest pH possible, while not compromise the product yield by undesired interaction with the membrane. The flowrate has been assessed as it might impact impurities binding by changing the retention time and the mass transfer (through size exclusion phenomena). The membrane loading factor has been tested until the maximum value recommended by the supplier.
3) Confirmatory study. The confirmatory study permitted to validate the DoE optimal condition.

Preliminary study and DoE analysis were realized using Ab1 (described in Example 1). The confirmatory study was carried out with Ab1 and Ab2, see details in Table 13. Ab2 was produced in HD-BIOP3 cells, which express the antibody high titers. The PA eluate for Ab2 was produced at 50 L pilot scale on a clarified harvest. Since higher titers may lead to an increase in process impurities levels to remove, the confirmatory study allowed to determine the robustness and the capacity of the developed platform.

**Table 13. Starting material**

| Antibody | pl | Cell line | Antibody titer | Antibody concentration in PA eluate |
|---|---|---|---|---|
| Ab1 | 8.13 | CHO-S | 1.5 g/L | 12.6 g/L |
| Ab2 | 8.41 | HD-BIOP3 | 6.1 g/L | 10.3 g/L |

Material and methods used for these studies are mainly described in Example 1. Additional information are given below.

### Viral inactivation

The targeted neutralization pH of the VI for the MA worst case preliminary study was 7.4. During the DoE three pH values were assessed (7.0, 7.2, 7.4), thus, three VI were done at the corresponding pH. The VI step was realized first with the acidification of a PA eluate to pH 3.5, the neutralization was done to 7.0 with the whole volume. For the two others target pH, the whole volume was split then neutralized to pH 7.2 or to pH 7.4, and some kept at pH 7.0. The VI for the confirmatory studies was done with a target pH of 7.2. Samples of PA eluate and VI neutralized were collected each time for product quality analysis by SE-HPLC, cGE and clEF as described in Example 1. The VI conditions for the different studies are reported in *Table 14.*

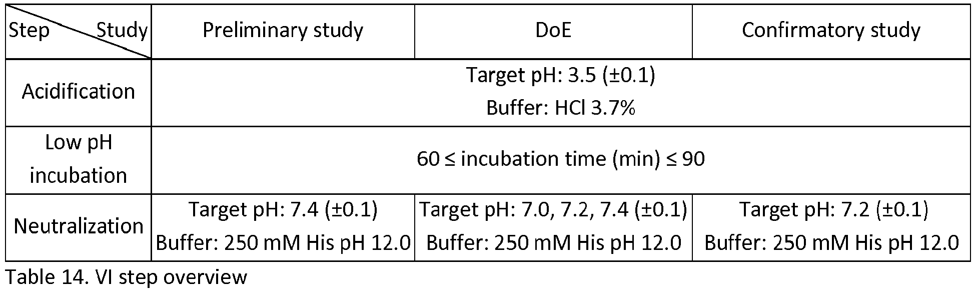

### MA step

The membrane used for all the runs of the study is the Natriflo™ HD-Q Recon Mini 0.2 mL from Natrix. The step of MA include:
1. Priming of the membrane adsorber with the equilibration buffer to remove the air from the membrane. The equilibration buffer used is different depending on the DoE condition tested (7.0, 7.2, 7.4).
2. Cleaning In Place (CIP) of the membrane to avoid microbial contamination of the membrane and system flowpath. CIP was done with 0.5 M NaOH, 2 M NaCl during five minutes at dynamic flow rate and then kept one hour in static mode to mimic manufacturing procedure.
3. Strip is done using 2 M NaCl to remove the hydroxide ions from NaOH for a shorter equilibration step.
4. Membrane equilibration carried out with the same buffer used for the priming.
5. Loading of the sample, namely of the VI neutralized sample, at the target loading factor (LF).
6. Flushing of the membrane with equilibration buffer to collect the dead volume.

### MA step - preliminary study

During the preliminary study the worst case conditions were tested. These worst case conditions include impurities load (i.e. maximum loading factor), membrane clogging due to air bubble or high flow rate which might lead to a pressure increase. The highest pH value was also tested as it is the limit of the buffer range provided by the supplier, above it precipitation could happen. Therefore, the VI neutralized sample concentrated at 11.5 g/L was loaded on the membrane adsorber to reach the maximum LF in supplier recommendation of 10 g_{Ab1}/mL_{membrane}. The maximum recommended flowrate (FR) of 25 MV/min was applied for the equilibration, load and flush steps, without pre-filter and with the flow restrictor in-line. At end the Histidine buffer pH was set at its range extremity, pH 7.4.

### MA step - DoE

### DoE set up

A full factorial Design of Experiment was set up and analyzed with the software JMP® 14.2.0 from SAS Institute. MA factors (inputs) were selected based on literature data and preliminary experiments. Three factors were taken in account for the optimization of the step: the loading factor, the pH of the load, equilibration and flush steps and the process flowrate (Table 15).

**Table 15 : Factors of the DoE, their unit, expected effect and different levels tested**

| | | Range tested (levels) | Unit | Effect expected on range tested |
|---|---|---|---|---|
| Factors (Inputs) | pH | 7.0/7.2/7.4 | n/a | Quadratic |
| | Flowrate | 5.0/25.0 | MV/min | Linear |
| | Loading factor | 2.5/5.0/7.5/10.0 | g/mL | Quadratic |

Three pH levels were chosen as the expected effect was a quadratic one. The pH value tested were 7.0, 7.2 and 7.4, in order to test the highest pH for Histidine buffer while staying in the capacity range.

Concerning the flowrate, two levels of 5.0 MV/min and 25 MV/min were tested and correspond to the range advised by the supplier. The LF levels were also set up following the supplier range recommendation. Moreover, as a quadratic effect was expected, due to a possible saturation of the membrane leading to an impurity breakthrough at high LF, 4 levels were evaluated. Values chosen were 2.5, 5.0, 7.5 and 10.0 g Ab/mL membrane.

All the DoE runs were done on Ab1. To minimize the responses variability due to the equipment, the MA were carried out on a single Aktä system. Samples were taken for each MA load and product for SE-HPLC, cGE, clEF analysis and HCP ELISA assay.

As the full factorial design screens the whole experimental space, the runs number is calculated by multiplying the number of level, so 3x4x2 = 24 runs. Three center points runs (CP) were added and correspond to the middle of the experimental space. They are essential to evaluate and integrate in the model the variability (pure error) of the method as they have the same factors level. If responses associated to the CP are too different, then the resulting model is not robust. Another important aspect of the DoE was the randomization of the runs order. It was performed by JMP and is presented in Table 16.

**Table 16 : List of DoE runs in the randomized order generated by JMP**

| **Run position** | **JMP pattern name** | **pH** | **LF** | **Flowrate** |
|---|---|---|---|---|
| 1 | 000 | 7.2 | 6.25 | 15.0 |
| 2 | 31- | 7.4 | 2.5 | 5.0 |
| 3 | 34+ | 7.4 | 10.0 | 25.0 |
| 4 | 34- | 7.4 | 10.0 | 5.0 |
| 5 | 32- | 7.4 | 5.0. | 5.0 |
| 6 | 14+ | 7.0 | 10.0 | 25.0 |
| 7 | 31+ | 7.4 | 2.5 | 25.0 |
| 8 | 11+ | 7.0 | 2.5 | 25.0 |
| 9 | 12+ | 7.0 | 5.0 | 25.0 |
| 10 | 21- | 7.2 | 2.5 | 5.0 |
| 11 | 23+ | 7.2 | 7.5 | 25.0 |
| 12 | 33+ | 7.4 | 7.5 | 25.0 |
| 13 | 21+ | 7.2 | 2.5 | 25.0 |
| 14 | 000 | 7.2 | 6.25 | 15.0 |
| 15 | 13- | 7.0 | 7.5 | 5.0 |
| 16 | 14- | 7.0 | 10.0 | 5.0 |
| 17 | 33- | 7.4 | 7.5 | 5.0 |
| 18 | 32+ | 7.4 | 5.0 | 25.0 |
| 19 | 13+ | 7.0 | 7.5 | 25.0 |
| 20 | 22+ | 7.2 | 5.0 | 25.0 |
| 21 | 22- | 7.2 | 5.0 | 5.0 |
| 22 | 23- | 7.2 | 7.5 | 5.0 |
| 23 | 11- | 7.0 | 2.5 | 5.0 |
| 24 | 24- | 7.2 | 10.0 | 5.0 |
| 25 | 24+ | 7.2 | 10.0 | 25.0 |
| 26 | 12- | 7.0 | 5.0 | 5.0 |
| 27 | 000 | 7.2 | 6.25 | 15.0 |

### Selection of the responses

The responses criteria to establish the optimum condition were chosen as presented in Table 17.

**Table 17 : Responses of the DoE, their unit in the model and the goal sought**

| | | **Goal** | **Unit** |
|---|---|---|---|
| **Responses (Outputs)** | Yield | Maximize | % |
| | HCP removal | Maximize | % |
| | IgG Monomer | Maximize | % |
| | Non-fragmented IgG | Maximize | % |
| | Main charged species | Match target value | % |

Important parameters to optimize were the yield of the MA step to maximize and the HCP level to minimize (or the HCP removal percentage to maximize). Additionally, monitoring parameters were included such as the IgG monomer purity to maximize (linked with aggregates minimization), the non-fragmented IgG percentage to maximize (linked with fragments minimization), and a main charged species percentage to match with a range between 40% and 50%.

### Analysis of the MA loads stability

The MA loads were re-analyzed before each MA run and HCP quantification was performed to study their evolution regarding product quality and process related impurities. If variation of the initial studied parameters had been observed in the MA loads through the time, the real step performance would have been false because not related to the same starting value. In this case a normalization of the HCP level would be required, using HCP removal percentage instead of absolute values in ppm.

### Responses distribution

Once the results were obtained, they were entered in JMP to set up the model. At first, the sampling population was analyzed for each response with the "distribution" tool of the software. It permitted to identify possible outliers and the distribution law fitted by the responses to interpret with robustness the statistical parameters described in the following section.

### Statistical parameters studied

For the model fitting, each response (yield %, IgG monomer %, non-fragment IgG %, main charged species and HCP removal %) was modelled individually from the others on JMP to optimize each response model robustness and significance. The software allows the use of many statistical parameters, tools and criteria but some of them were specifically useful for the analysis.

The first one is the lack-of-fit p-value, if it is superior to 0.05, it means that the model generated fits enough the observed response to explain and predict it. It also means that the total error of the model is mainly explained by the variability of the method, not a weak fitting of the model. Then, the Analysis of Variance (ANOVA) p-value, if inferior to 0.05, means that the average response associated to different levels of a factor are different. As an example, a significant ANOVA p-value for the yield would mean that the average yield associated to pH 7.0 is different than for the 7.2 one and the 7.4 one and so that the levels of a factor have an impact on the response associated. The Akaike Information Criterion (AICc) was largely used as it gives information on the model robustness. The smaller this criterion is, the better the model is. Effect estimates and p-values were also studied, if an effect p-value is below 0.05 it means that this effect is significant for the response. The estimate represents the influence of the factor on the response. During the improvement of the models, non-significant factor effects were successively removed to attempt to minimize the AICc, meaning that the models were better. At end the Rsquare value was a simple indicator of which percentage of the observed response is explained by the model generated. The higher the Rsquare is the better it is. A small Rsquare does not mean that the model is wrong but that the factors selected and the model explain a small percentage of the empirical response.

### MA step - Confirmatory study

The two molecules, Ab1 and Ab2 were used. MA was performed according to the following conditions: equilibration, load and flush were done at pH 7.2, 10 g/L of LF and 25 MV/min of FR, which were the DoE determined settings. The runs were processed on the same Aktä system with within 24 hours. This time the MA were performed within the two days following the VI, so it was considered that the VI neutralized equalled the MA loads. MA products samples were collected for routine, HCP and DNA analysis. Table 18 summarizes the different MA steps realized and the conditions associated.

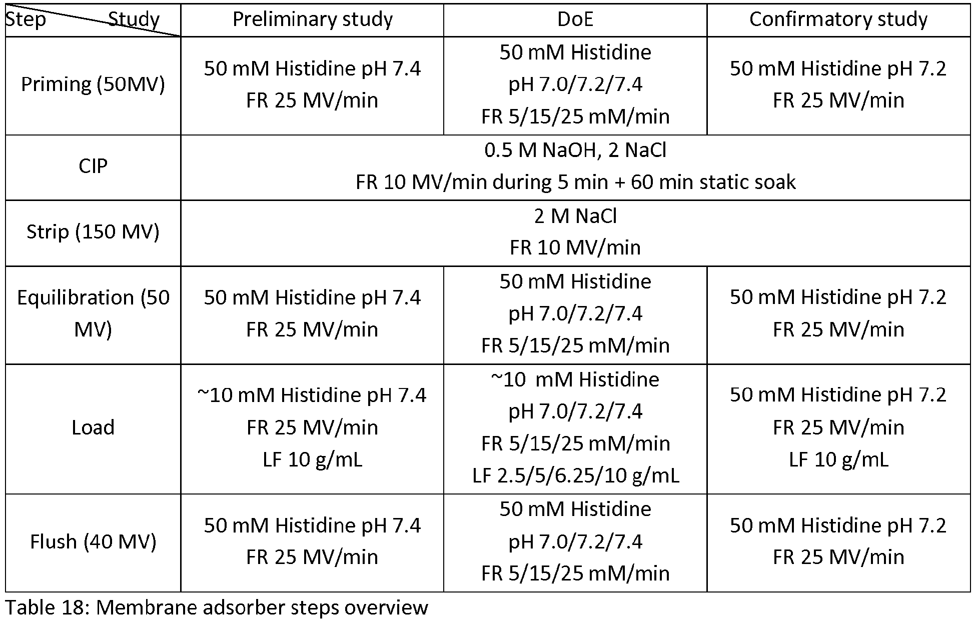

### Cation exchange chromatography

CEX in bind/elute mode mainly removes aggregates and different impurities such as mAb fragments, protein A leachables, some viruses but also HCP and DNA. CEX resin is the Poros™ XS from Thermofisher.

In this study, the CEX was performed during the confirmatory runs, after the MA step to evaluate the performance of the whole MA - CEX sequence compared to current CEX - UFDF1 - MA one.

To perform CEX, MA products were adjusted with 1 M Citrate pH 3.5 to pH 5.5 to fit with the loading conditions of the CEX step. As the CEX load corresponds to the MA product adjusted, samples were taken of this new intermediate to assess the impact of the pH adjustment on product quality and HCP / DNA levels.

Prior to start the run, the resin was CIP with 0.5 M NaOH, and equilibrated in 25 mM Citrate pH 5.5. The product was then loaded at maximum LF of 45 gmAb/Lresin considered as a worst case scenario for impurities removal. Then a wash was realized to remove the impurities with a low affinity for the resin with 25 mM Citrate 40 mM NaCl pH 5.5 for Ab2 and 25 mM Citrate 20 mM NaCl pH 5.5 for Ab1. The isocratic elution of mAbs was performed with 25 mM Citrate 100 mM NaCl pH 5.5 for Ab2 and 25 mM Citrate 60 mM NaCl pH 5.5 for Ab1. Another difference between the two Abs is that the eluate collection for Ab1 starts when the absorbance is above 50 mAu/mm (2 mm UV cell) and last 13 Column Volume (CV). For Ab2, the eluate collection starts when the absorbance is greater than 100 mAu and finishes when it goes down 100 mAu. To finish a strip is done with 2 M NaCl, a CIP with 0.5 M NaOH and the column is stored in 0.1 M NaOH. The flowrate was fixed at 400 cm/h for all the steps except the column storage, at 200 cm/h. As for all the process intermediates, CEX eluate samples were withdrawn for analysis presented in section 4.3.6, HCP and DNA analysis. Table 19 summarizes the different CEX realized and the conditions associated.

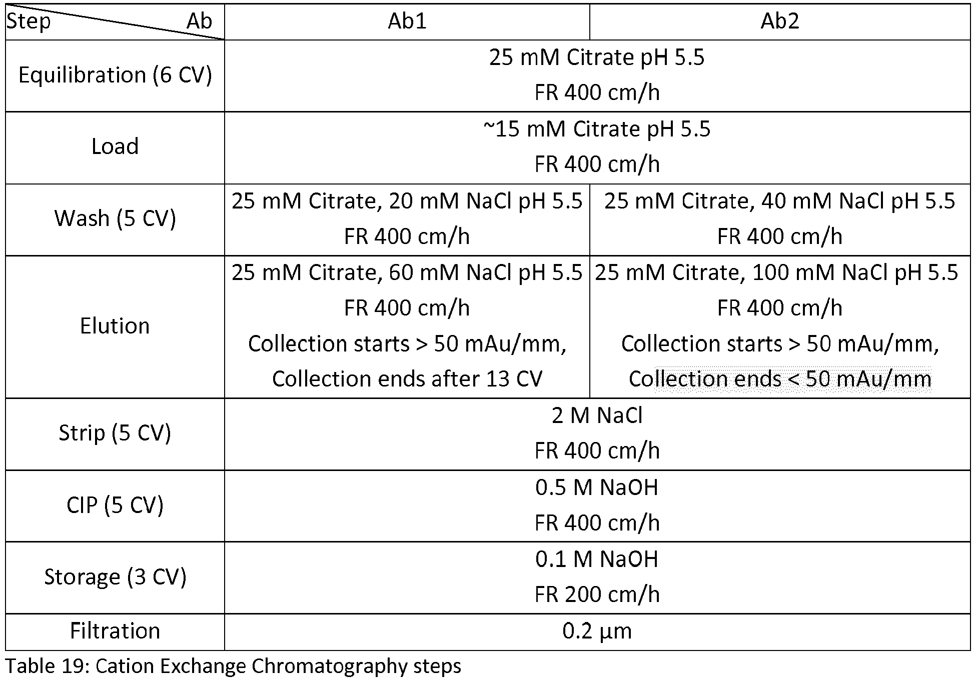

### HCP and DNA analysis

### ELISA HCP assay

The HCP quantification was performed with the 3G ELISA HCP kit of Cygnus technologies that covers about 30% of CHO cells HCP. The samples were reacted with an affinity purified horseradish peroxidase labelled antibody, in microtiter wells coated with an anti-CHO HCP capture antibody. Following washes, the substrate tetramethyl benzidine was reacted. The amount of hydrolyzed substrate was directly proportional to the concentration of HCP protein present. The quantification was achieved by comparing the signal of samples to HCP standards assayed at the same time. The plate reader used was SpectraMax® i3X from Molecular Devices with the software Softmax Pro 7.0.3.

All the samples were quantified in duplicate. The positive and negative controls were furnished in the Elisa kit. The quality of pipetting was also verified by spiking HCP from the kit in each sample in duplicate. As the absorbance measured needed to fit in with the standard range, dilutions were realized, based on previous HCP quantifications and are described in Table 20.

**Table 20 : Dilutions for HCP Elisa assay**

| Process intermediate | Study section concerned | Dilution assayed |
|---|---|---|
| PA load | Confirmatory runs | 1/60000 |
| | | 1/30000 |
| | | 1/10000 |
| | | 1/1000 |
| PA eluate | DoE and Confirmatory runs | 1/100 |
| VI neutralized | Confirmatory runs | 1/40 |
| MA load | DoE runs | 1/40 |
| MA product | DoE and Confirmatory runs | 1/10 |
| CEX load | Confirmatory runs | 1/10 |
| CEX eluate | Confirmatory runs | 1/5 |

### DNA analysis

DNA analysis involves a treatment of the samples by the proteinase K to remove proteins. Then, DNA is extracted using magnetic beads, washed and finally eluted. DNA is quantified by a real-time PCR using Fast 5000 PCR equipment (Applied Biosystem). Samples preparation were done with resDNASEQ™ CHO DNA Kit V3.0 (Applied Biosystem).

### Example 3: Optimization of the MA step for PP2 - preliminary study results

The preliminary study was carried out to test the worst case parameters (pH 7.4, LF 10 g/mL, flowrate 25 MV/min) as described in Example 2.

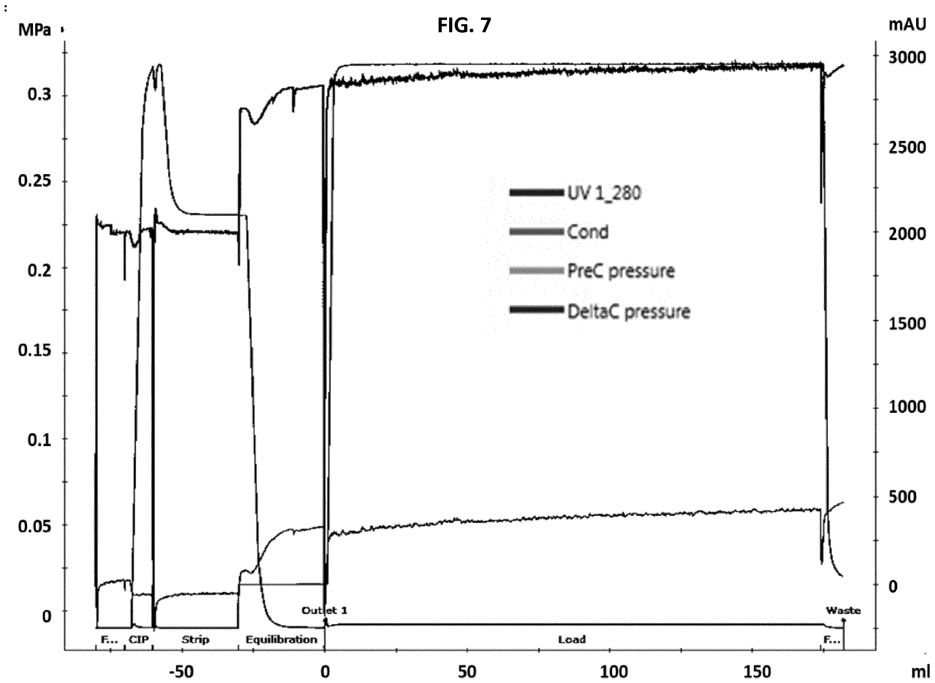

As previously described, a VI was done on a Ab1 PA eluate to produce the material to assess the MA step with these parameters. During the MA, the maximum pressure observed on the membrane was 0.3 MPa, which is inferior to the 0.5 MPa maximum handled by the membrane (Figure 7). The step yield was calculated at 100% (Table 21). The test allowed us to cover and validate the whole factor ranges that should be tested in the DoE runs.

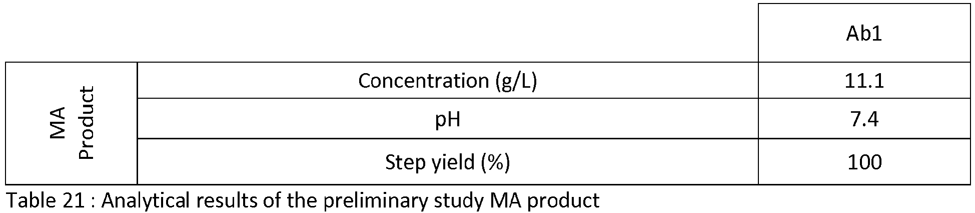

This preliminary study showed that the membrane can handle challenging conditions, in term of processing settings but also as a first polishing step post Protein A and VI steps. Then, the VI neutralized product could be processed fastly, while maintaining high yields and HCP removal.

### Example 4 Optimization of the MA step for PP2 - DoE results

### Viral inactivation steps

The viral inactivation steps of the PA eluates permitted to produce the MA loads, material necessary to realize the DoE MA runs of the study.

The same initial material as the preliminary studies previously described was used and the analytical results are presented in Table 22.

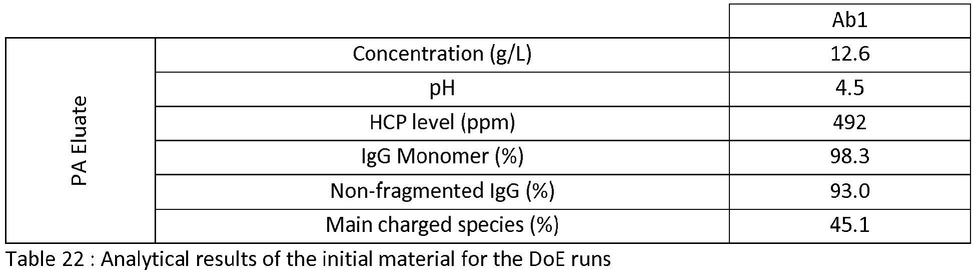

A precipitate was observed since pH 6.0 was reached, and more intense at pH 7.4 than pH 7.0. (Table 23).

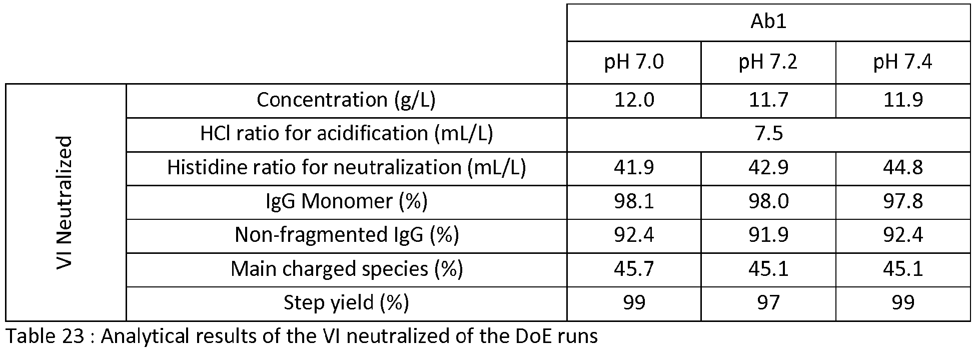

The acidification was done by adding 7.5 mL HCl 3.7%/L_{Ab1}. Regarding the neutralization at pH 7.0, 7.2 and 7.4, the Histidine volumes added were respectively 41.4, 42.9 and 44.8 mL_{250 mM His} /L_{Ab1}. An average molarity of 10 mM Histidine was added. Introduction of Histidine is desired to put the product in a matrix suitable for the MA step. The three steps yields were calculated above 97%. The routine analysis of product quality on VI neutralized gave usual results.

The precipitation during the neutralization seemed to not alter the product quality, as the variation observed is inferior to 1% between the PA eluate and the VI neutralized for all the analysis. All the analytical results were within expected ranges and comparable to results observed in RPP with the same product (> 97.5% of monomer and > 92% of non-fragmented IgG). This observation was done after a seven-week study, proving that no product degradation happened during this time.

### MA loads analysis

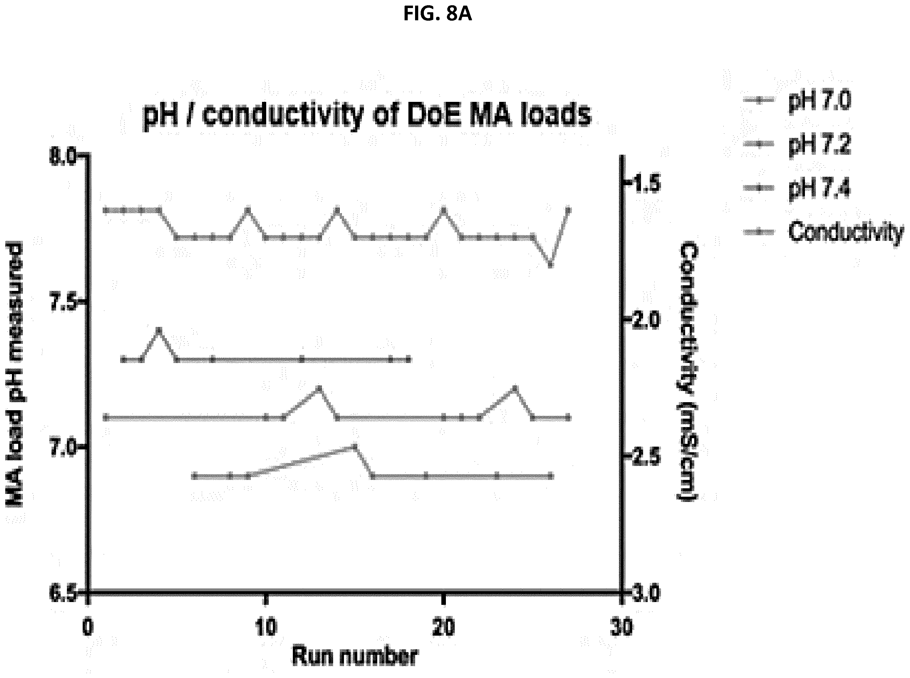

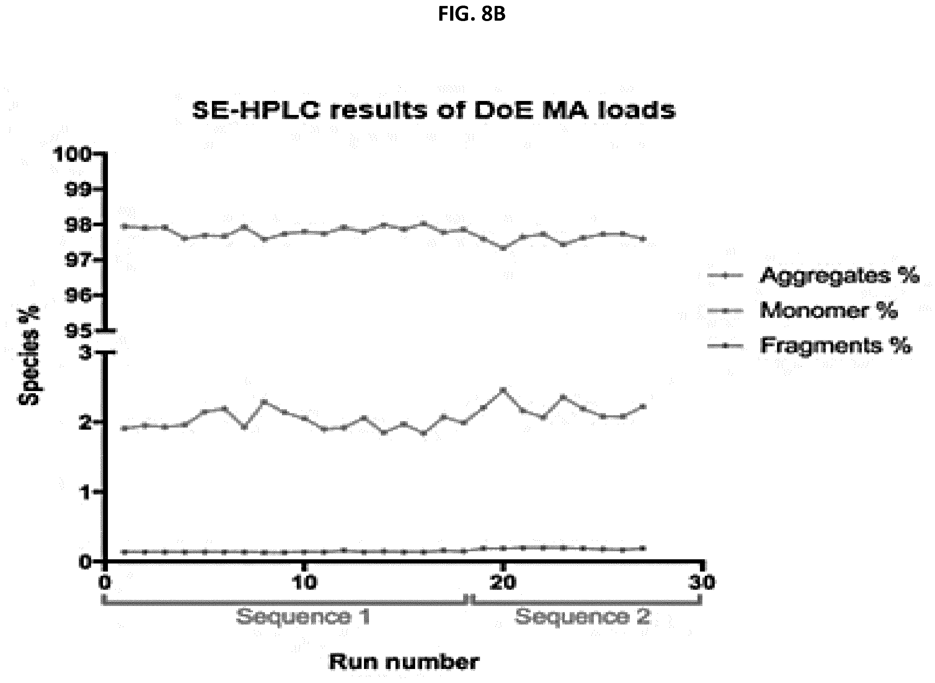

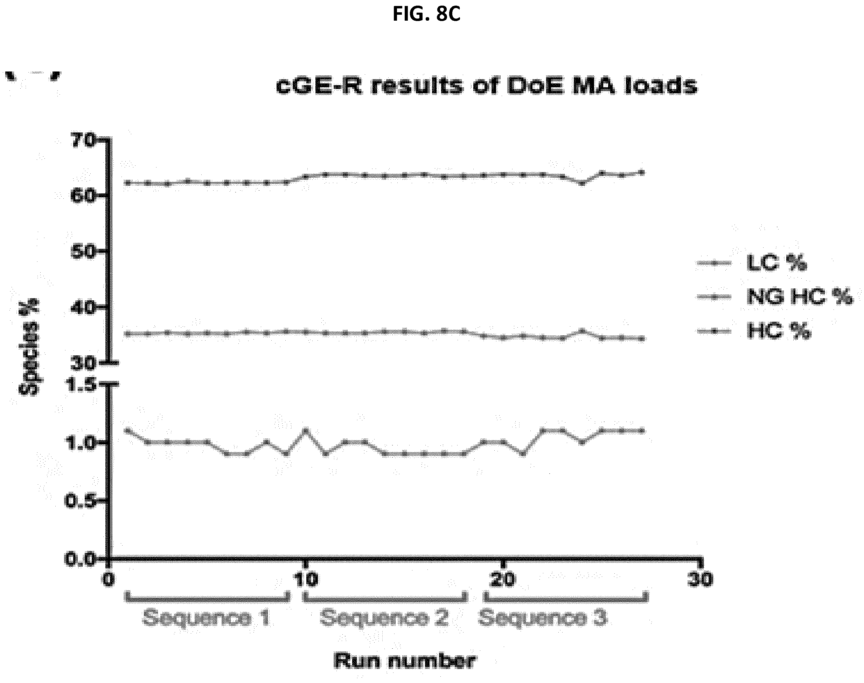

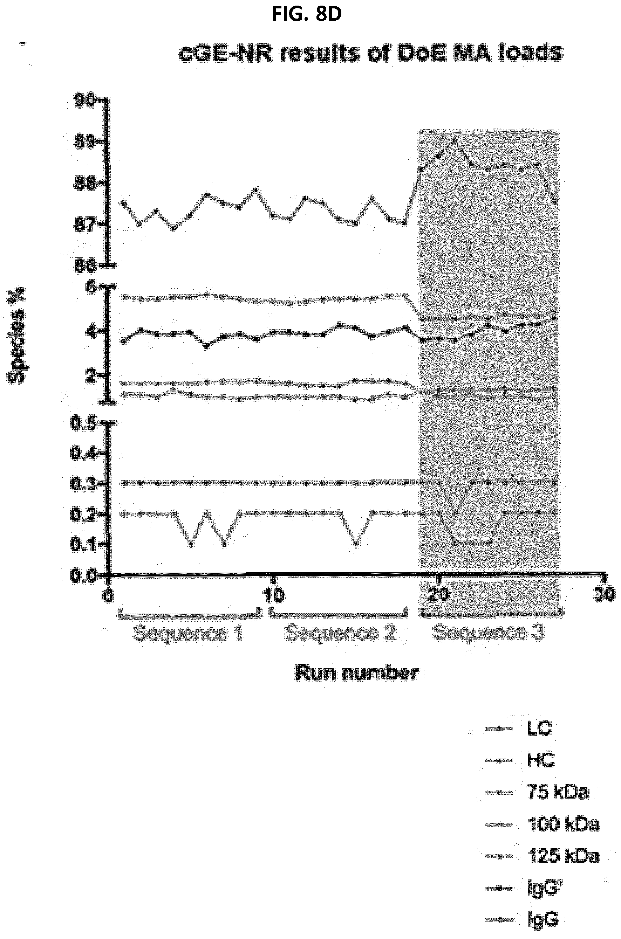

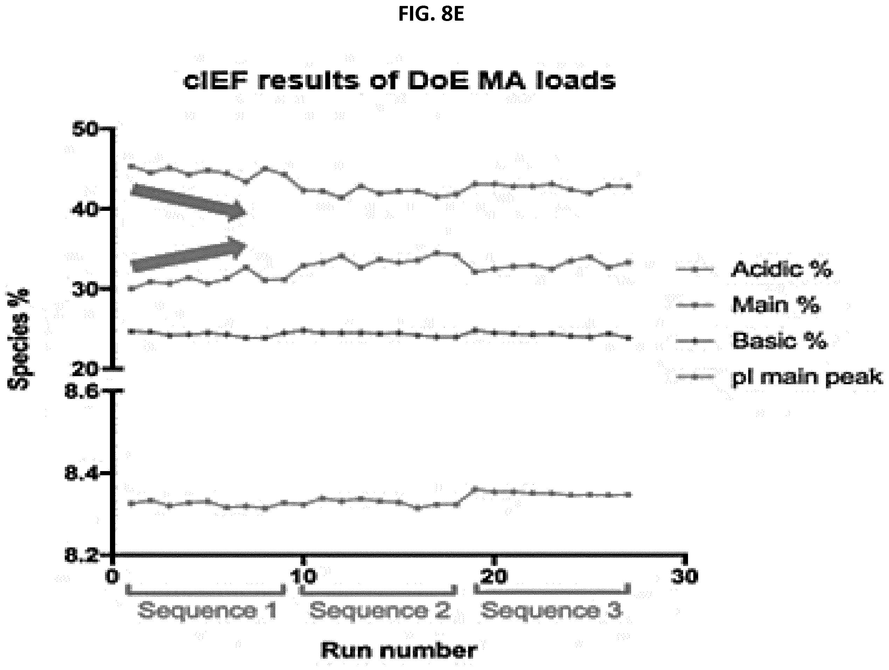

The MA load analysis was done to identify trends in the responses that could be linked to the duration of the study and which could impact the DoE modelling. The MA loads over the DoE period showed no atypical variationsof the pH or conductivity (Figure 8a). The same observation was done for IgG monomer (SE-HPLC), HC/LC ratio (cGE-R) and the main charged species, even if results showed a slight increase of acidic species over time (Figure 8b, c and e). Regarding the non-fragmented IgG percentage, an increase of about 1% was observed. However, the results which showed higher percentages were all in the third analytical sequence of analysis. Moreover, less than 24 hours separated the last run of the second sequence and the first of the third one, thus, it is unlikely to have a degradation within this duration. Therefore, the difference was probably not due to the condition but to the analytical method sequence (Figure 8d).

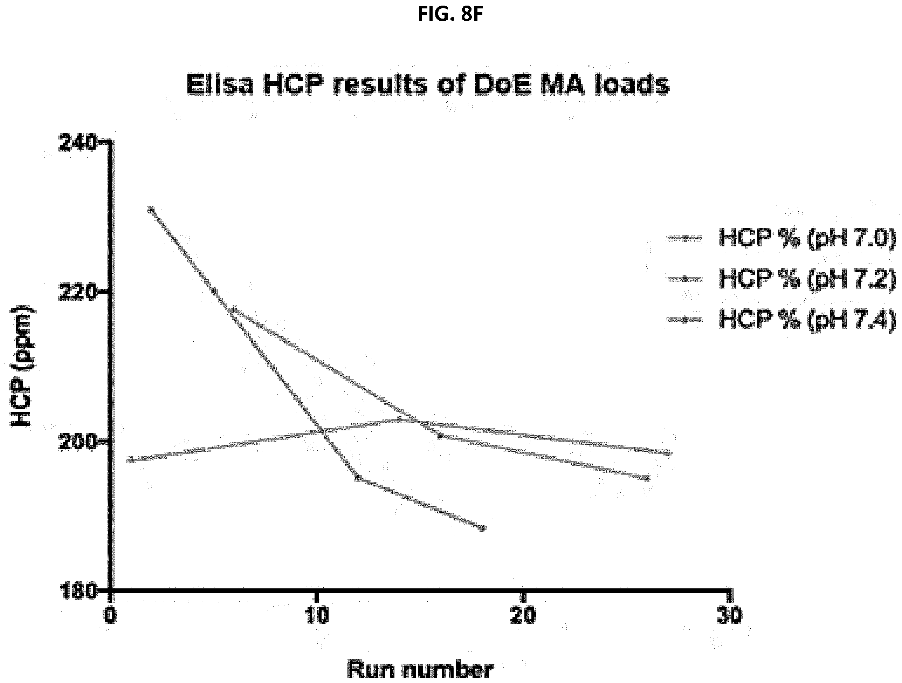

As noticed during the VI step, a white precipitate appeared gradually in the VI neutralized bottles and more intensely at pH 7.4 than 7.0. The HCP Elisa assay have shown a decrease in the HCP level at pH 7.4 with a loss of 40 ppm (Figure 8f). Results at pH 7.0 showed a weak decreasing trend and no significant evolution of the MA load has been identified at pH 7.2 compared to pH 7.0 or 7.4. In summary, the MA load observations showed that the 10 mM Histidine added during the VI processing are sufficient to maintain the product at the desired pH over several weeks. It showed that the analytical methods could introduce variability in the results between analysis sequences. The VI pH has a proven impact on the process impurities precipitation but also the storage duration. Even if a PA eluate should not be stored more than some days before being processed, it has been a crucial information to determine how to input the results in the DoE. That is the reason why it appeared important make DoE analysis with percentage of HCP removal instead of absolute values in ppm, as the initial amount in the MA load varied over time. Consequently, the percentage for every run was calculated using the closest HCP MA load value in the time period of a run, to have the correct estimate of the HCP removal.

### DoE runs and response distributions observation

Prior to model the responses assessed in the DoE, the data and their distribution were observed to observe the data dispersion and the robustness of the resulting model.

### DoE runs analytical results

The average results of the 27 runs are presented in Table 24. The response which varied the most is the HCP removal percentage (from 82% to 90%), and over the variability of the ELISA test (1 to 2 ppm). Regarding the other responses, the IgG monomer percentage varied between 97% and 98%, the non-fragmented IgG percentage between 91% and 93%, the main charged species between 43% and 45% and finally the yield between 96% and 99%.

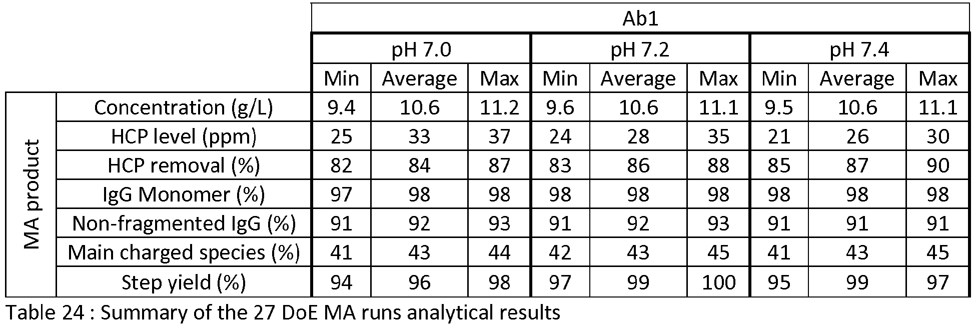

Thus, no significant difference was observed between the minimum and the maximum values obtained for each analytical response. Regarding the MA products concentration, the difference are due to the LF. The HCP, and so likely other process related impurities, seemed to be better eliminated at pH 7.4 as expected with the previous observations. The first conclusion has been the generally good quality of the results despite the use of high flowrate and LF. The entire experimental space of the DoE is comparable to historical data in term of HCP removal, IgG monomer percentage and step yield, and showing high robustness of the process over the studied range. However even though the differences were slight, trends were identified for the pH which was not the case for the LF and flowrates. Once trends were identified, their distribution have been analysed in JMP.

### Statistical population distributions

The distribution of the responses population was realized prior to the modelling. This allowed to identify the statistical trend of the responses and possible outliers to eliminate.

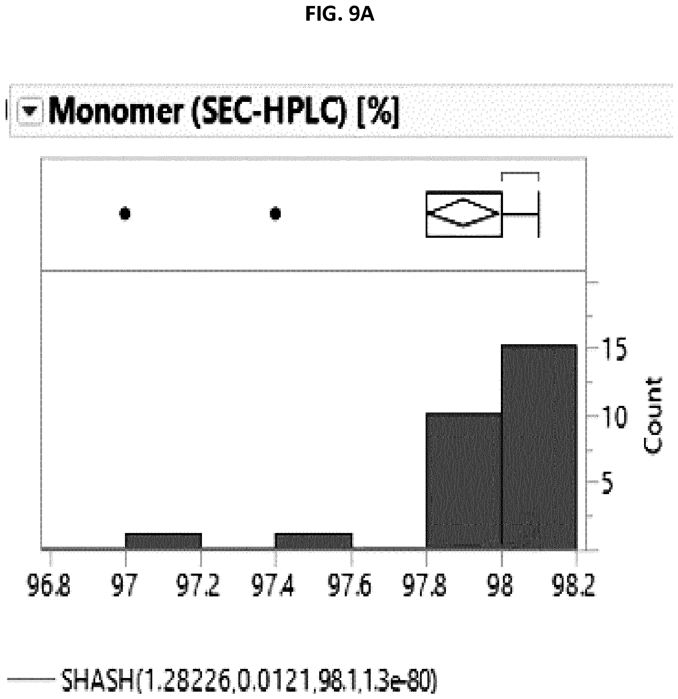

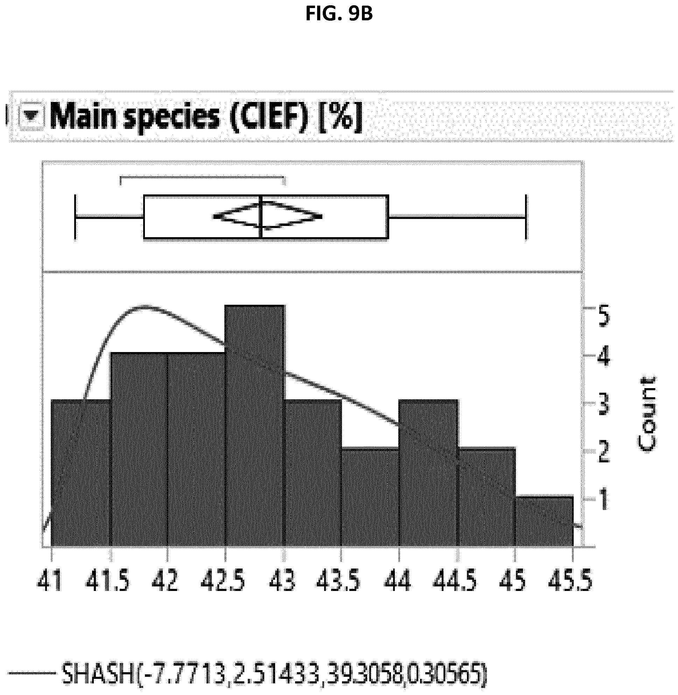

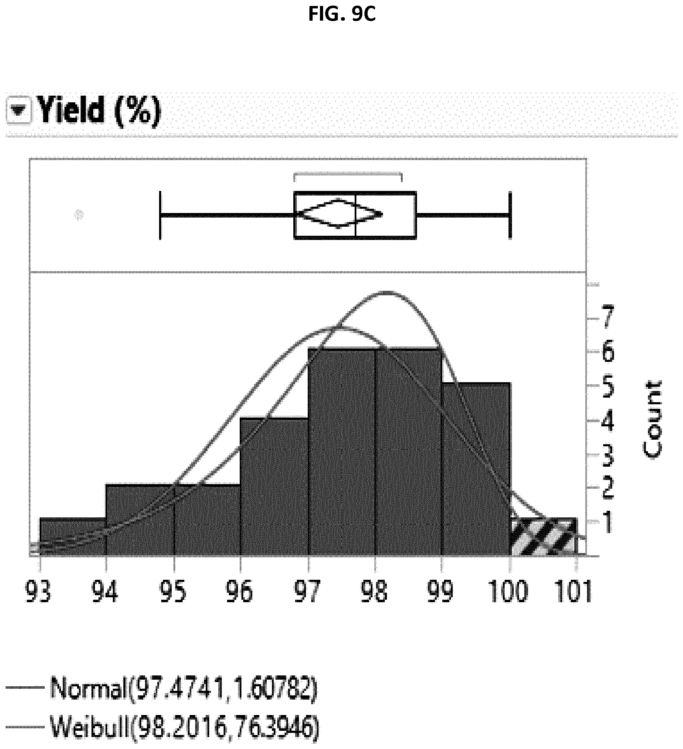

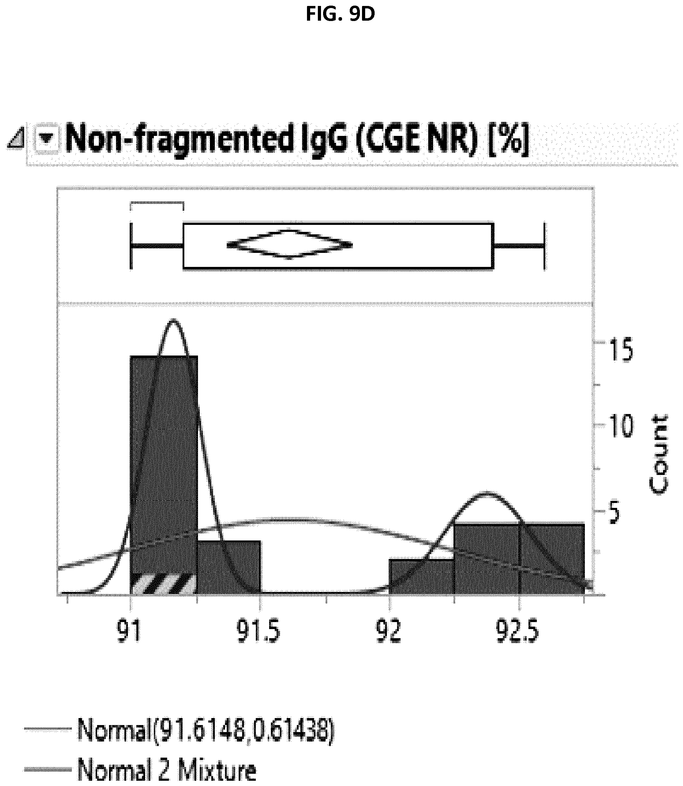

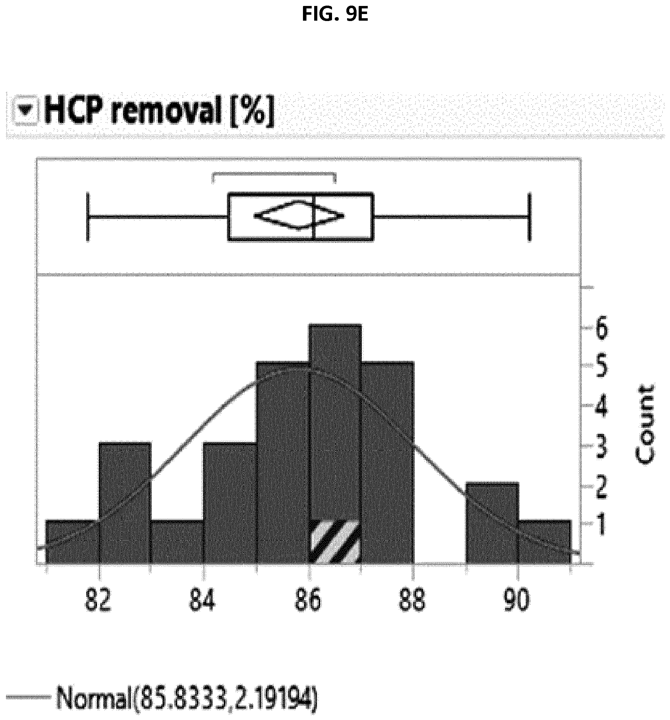

The IgG monomer percentage and the main charged species followed a SHASH distribution (Figure 9a, b), the yield a Weibull distribution (Figure 9c), and the HCP removal a normal law (Figure 9e). The non-fragmented IgG percentage was a two-mixture normal distribution (Figure 9d) but value groups correspond to the analytical sequences. The quantile box plot showed two potential outliers for the IgG monomer response and only one for the yield response.

The distribution analysis led to the identification of the responses that did not follow a normal distribution (Monomer %, non-fragmented IgG %, and main charged species %). Most of the statistical criteria used by the software are based on Student and Fischer tests, which are preferably applicable to normal distributions. The yield and HCP removal responses almost followed a normal distribution and could therefore be treated with confidence in JMP. For the other ones, it was still possible to analyse them in JMP, as the most important thing is the independence of the experiments. The conclusion has been that IgG monomer, main charged species and non-fragmented data should be examined with more attentiveness and critical mind-set. From a scientific perspective, these responses should fit to a normal distribution but the variability of the method and the small population size led to these results. At this point, the models fitting could have been possible to analyse the responses and remove if necessary the outliers identified.

### Response modelling and prediction of optimal condition

The final step of the DoE study has been the model fitting of the responses and the use of the prediction profiler tool to determine the optimal condition. It also provided the prediction of the responses values associated to these conditions.

### Model fitting of responses separately

As presented before, the responses were modelled separately and analysed thanks to different statistical estimators. All these parameters give information about the robustness of the model and the significance of the responses (Table 25). It helped to decide if some responses should not be taken into account, in order to determine the optimal desired condition.

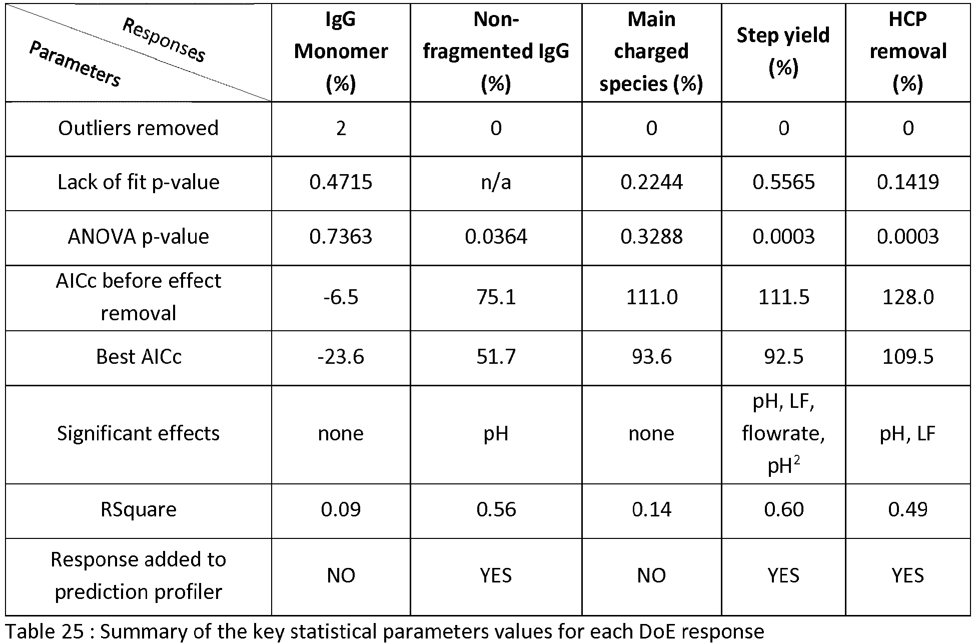

During the modelling, it was necessary to remove two outlier from the IgG monomer response. Indeed, keeping these outliers demonstrated that monomer % was a significant response, i.e., monomer % was impacted by some of the factor studied. However, based on process knowledge, MA step is not intended to remove aggregates (they are flowing through together with the monomer). As no impact were expected for this response and the initial distribution for this response was not normal, it was decided to remove the outliers. This removal led to a positive ANOVA, which excluded the response and allowed to make a better model to find the right optimal process condition. Moreover, the AICc associated decreased, which showed a better robustness of the model. All the model lack-of-fit p-values were above 0.05, thus the generated models fit with the observations. The yield and HCP removal responses have ANOVA p-value fully inferior to 0.05, the non-fragmented IgG response is close to the non-significance level (0.3288), and the IgG monomer and main charged species responses are above 0.05.

For the IgG monomer response and main charged species, the ANOVA analysis means that the different levels of factors tested have no impact on the response. In other words, the factors tested are not the good ones to optimize these responses. Moreover, the non-fragmented IgG response was weakly impacted by the studied parameters but was kept for the following prediction profiler. This result was also expected as the MA is not intended to remove fragments neither. Finally, after polishing the model by removing non-significant effects, the ones remaining are the pH for the non-fragmented IgG response, then the LF, flowrate and the quadratic effect of pH (pH²) for the yield response, eventually the pH and LF for the HCP removal response. Those three responses were used to set up the prediction profiler of the MA step regarding the pH, LF and flowrate of the step.

### Prediction profiler

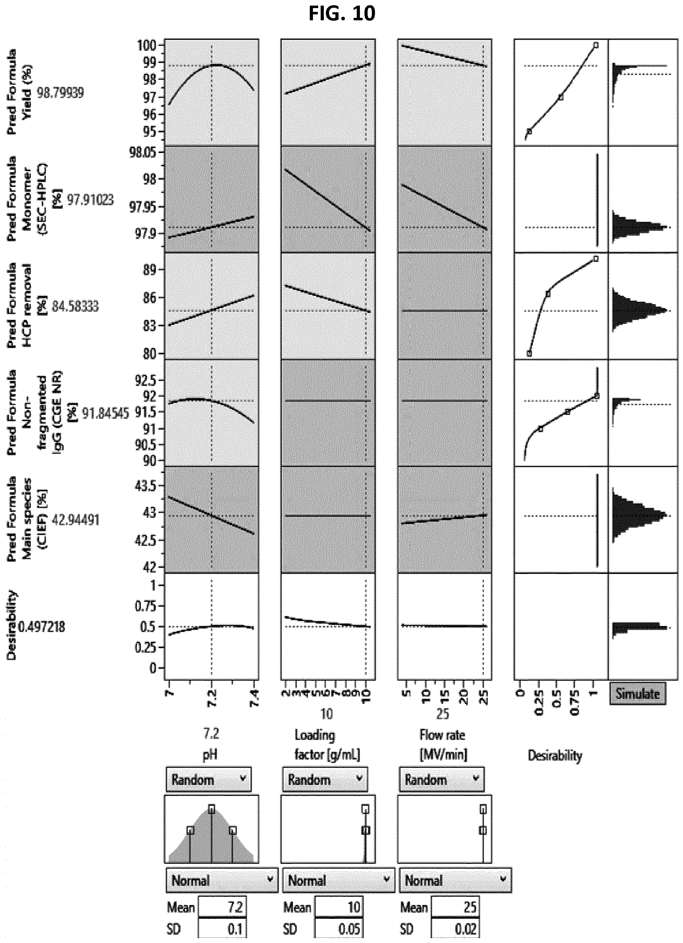

The last step of the DoE is the interpretation of the prediction profiler (Figure 10). Each model is an equation that predicts the response variation associated to the factors value and their effects. Even if the response were modelled separately it is important to analyse it together with the profiler as they are part of a system as a whole. As it has been decided to not include the IgG monomer and Main charges species as significant responses in the profiler, the desirability associated with these responses was set to null value. The HCP removal desirability was set to three and the others at one, as the main goal of this optimization has been to focus on process related impurities removal.

The predicted yield varied between 94.8% and 100% on the entire factor ranges, the HCP removal between 82.8% and 88.8% and the non-fragmented IgG between 91.1% and 92%. We also confirmed the previously identified trends. The quadratic effect of the pH on the yield (2% amplitude), was observed with an optimum between pH 7.2 and 7.3. The yield also increased when the LF increased (2% amplitude) and the flowrate decreased (1% amplitude). For the HCP removal percentage, higher was the pH and lower was the loading factor, better was the HCP removal (3% amplitude for both). Finally, the effect of pH on non-fragmented IgG response was low but the IgG seemed to be lower with more basic pH values (0.7% amplitude).

The standard deviation linked to the equipment was added to the profiler, in order to have an estimation of the response distributions around the predicted one (illustrated by a histogram) to mimic the manufacturing scale conditions. The value was set at 10% for the pH as it is the method variability of the pH-meter. The LF standard deviation at 5% linked to the Nanodrop for the concentration measurement. At end the flowrate variability was set at 2%, the one of the Äkta pumps. It was noticed that the pH is the most critical factor to control, as a small variation of its value would lead to a significant variation in the responses, mainly for the HCP removal. Thus, it should be taken into account for the choice of the optimal pH value for MA step at manufacturing scale even if in all cases the HCP removal remains high.

To conclude, even if trends were identified for the significant responses of the prediction profiler, their variation remained small for the factor levels evaluated. However, the responses were in the range generally observed for a MA step and the performance was high for each response. The whole levels tested gave excellent results, which allowed to choose the optimal MA condition not only regarding the product quality but also the process productivity and robustness.

### Optimal condition determination

Using the profiler, factors or responses were modified according to the objectives, whether the best product quality or the best productivity was chosen. Two options were simulated in the prediction profiler, first the optimal settings for HCP removal, then the optimal ones for productivity (Table 26). The results were pH 7.4, LF 2.5 g/mL and flowrate 5 MV/min for a better HCP removal, and pH 7.2, LF 10 g/mL and flowrate 25 MV/min for a better productivity.

**Table 26 : Summary of the scenario, the factor values associated and the responses predicted**

| | pH | Loading factor (g/mL) | Flowrate (MV/min) | Yield (%) | IgG Monomer (%) | HCP removal (%) | Non-fragmented IgG (%) | Main charged species (%) |
|---|---|---|---|---|---|---|---|---|
| Optimize HCP removal | 7.4 | 2.5 | 5 | 96.9 | 97.9 | 88.7 | 91.2 | 43.5 |
| Optimize productivity | 7.2 | 10 | 25 | 98.8 | 97.9 | 84.6 | 91.8 | 42.9 |
| Difference productivity / HCP removal | -0.2 | 7.5 | 20 | 1.9 | 0 | -4.1 | 0.6 | -0.6 |

Concerning the productivity, a high flowrate would allow to process the material faster and a high loading factor to minimize the number of membrane and buffer to use. This could be an interesting aspect with the development of cell lines that express Abs at high titer. The pH 7.2 condition is preferable because it allows a high HCP removal while balancing the variability of buffer preparation (± 0.1 pH unit). In this condition the responses were expected around 98.8% for the yield, 84.6% for the HCP removal and a non-fragmented IgG level at 91.8% for the MA step. Despite it has been shown that the main charged species and monomer % responses were not significantly impacted by the MA step, the prediction profiler was predicted to be 42.9% and IgG monomer at 97.9%.

Compared to the condition for the best HCP removal, a better yield is obtained with this settings (98.8% instead of 96.9%) and an efficient HCP removal (84.6% instead of 88.7%). The difference in term of HCP removal represents only 6 ppm but using this condition, allowed faster processing than in the other settings. In addition, a part of the remaining HCP is supposed to be removed by the following CEX and there is consequently a safety margin for the HCP removal. To conclude, the MA setting chosen for the PP2 were pH 7.2, 10 g/mL LF and flowrate 25 MV/min (Table 27).

**Table 27. Optimized MA steps**

| **Step** | **Parameters** |
|---|---|
| Venting (50 MV) | 50 Mm Histidine, pH 7.2 |
| | FR 25 MV/min |
| CIP | 0.5 M NaOH, 2 NaCl |
| | FR 10 MV/min during 5 min + 60 min static soack |
| Strip (150MV) | 2 M NaCl |
| | FR 10 MV/min |
| Equilibration (50 MV) | 50 M Histidine pH 7.2 |
| | FR 25 MV/min |
| Load | ^{∼}10 M Histidine pH 7.2 |
| | FR 25 MV/min |
| | LF 10 g/mL |
| Flush (40 MV) | 50 mM Histidine pH 7.2 |
| | FR 25 MV/min |

### Example 5: Optimization of the MA step for PP2 - confirmatory studies results

Once the optimal condition has been determined by DoE, they have been confirmed using Ab1 and Ab2 as described in Example 2. The characteristics of PA eluate containinf Ab1 or Ab2 are reported in Table 28.

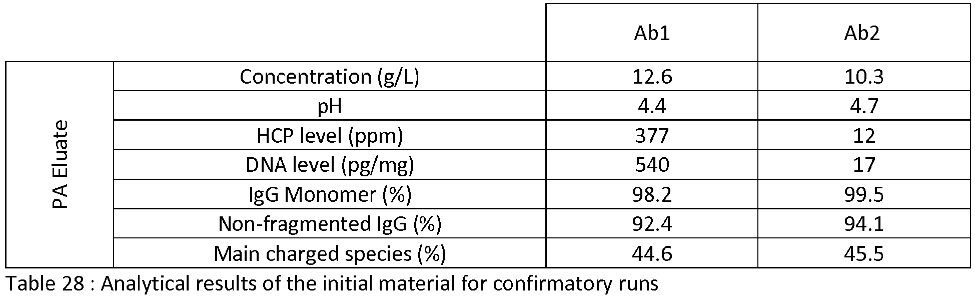

### Preparation of Ab1 and Ab2 material for MA - CEX steps

The VI step allowed to produce the material for the MA - CEX runs in order to compare the obtained results with the previous platform performance. As previously, the acidification step of the VI was realized to pH 3.5 and the neutralization was then done to pH 7.2 to fit with the MA loading conditions (Table 29).

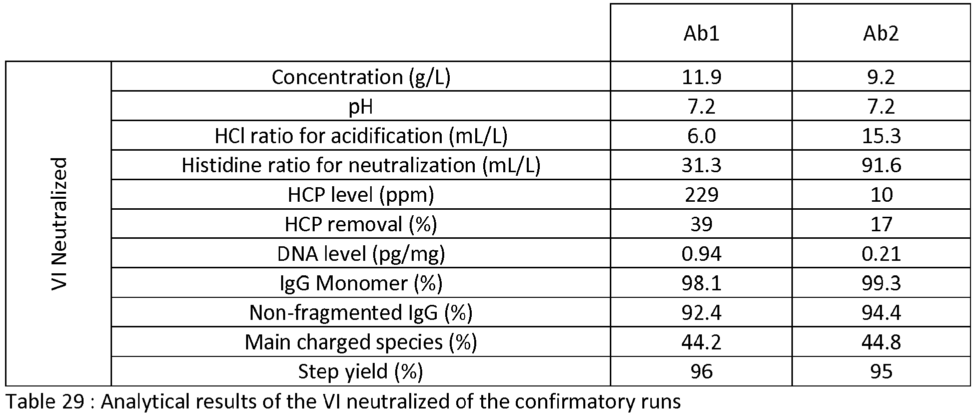

The initial HCP levels were about 377 ppm for the Ab1 PA eluate material instead of 12 ppm in the Ab2 eluate, meaning 30 time fold higher. The same observation was done with the DNA level, with 17 pg/mg in the Ab2 PA eluate and 540 pg/mg in the Ab1 one.

As observed in previous experiment, a precipitation was observed for Ab1 during the VI step. However, no precipitates were observed for Ab2 neutralization even at pH 7.2. The acid ratios were 6.0 ML_{HCL3.7%}/L_{Ab1} and 15.3 mL_{HCL 3.7%}/L_{Ab2}, for the neutralization were added 31.3 mL_{250 mM His}/L_{Ab1} and 91.6 mL_{250 mM His}/L_{Ab2}. These volumes represent an addition of 7.6 mM of Histidine in Ab1 product and 21.0 mM in Ab2 one. For the Ab1 VI, the yield was calculated at 96% and a 39% of HCP removal. Regarding Ab2, the yield was of 95% with a 17% of HCP removal. The HCP level was much lower in the Ab2 VI neutralized (10 ppm) than the Ab1 one (229 ppm). However, this observation is linked to the difference in the HCP level of the initial PA eluate materials. The VI step removed most of the CHO cells' DNA with post step levels of 0.94 pg/mg (Ab1) and 0.21 pg/mg (Ab2). The routine analysis on VI neutralized gave very promising results with Ab1 (98.1% IgG monomer, 92.4% non-fragmented IgG and 44.2% main charged species) as well as with Ab2 (99.3% IgG monomer, 94.4% non-fragmented IgG and 44.8% main charged species).

It seemed that Ab2 VI load was less sensitive to the acid / base addition as more volume was needed. Consequently more Histidine was introduced, for a better buffering effect without diluting too much the product. The Ab1 product quality was good and comparable to previous results obtained after VI. The same observation was done with Ab2 results which are comparable to historical data of this molecule. The VI step allowed to well remove DNA in both case, reaching levels below 1 pg/mg.

### Optimized MA step implementation

No overpressure was observed for the two MA runs and the yields obtained were both of 100%. The HCP removal was measured at 86% for Ab1 and 90% for Ab2 which represent respectively 32 ppm and 1 ppm (Table 30). The DNA levels were measured at 0.14 pg/mg (Ab1) and 0.19 pg/mg (Ab2). The product quality measured was almost the same as the VI neutralized one for Ab1 (98.1% IgG monomer, 92.1% non-fragmented IgG and 45.0% main charged species) and Ab2 (99.2% IgG monomer, 94.2% non-fragmented IgG and 44.4% main charged species).

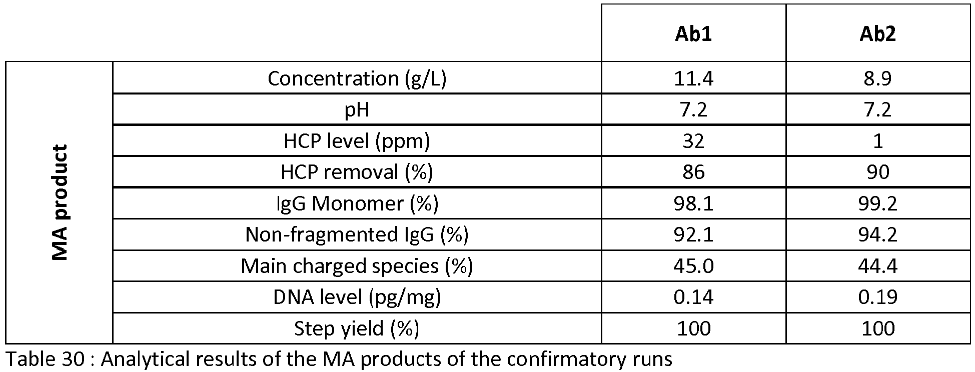

First of all it was noticed than the optimization of the MA step was done well as the results were nearby or better than the predictions of the model in term of yield, HCP removal and product quality. Moreover, both MA products were in the expected range for product quality, HCP level (< 30 ppm) and DNA level despite the differences between the two mAbs. Thus, the MA step seemed robust in this case, and the performance of the CEX needed to be determined to fully compare the fully polishing package (i.e. MA - CEX) of the optimized platform with the previous one.

### The CEX as a second polishing step

A CEX step was done on Ab1 and Ab2 to obtain the data for all the polishing steps of the platform and compare it. The goal was also to prove if the CEX remained an efficient step to remove fragment and process related impurities even after the MA step optimized.

The pH adjustment was done with 14.5 mL_{1M Citrate}/L_{Ab1} and 25.1 mL_{1M Citrate}/L_{Ab2} (Table 31). During the runs no overpressures were signalled, but a breakthrough of Ab2 was observed during the wash step. The final concentrations in the eluates were 7.3 g/L for Ab2 and 3.0 g/L for Ab1, the latter being more diluted due to the collection criteria presented before. The yield for the Ab1 CEX was calculated at 87% and 81% for the Ab2 one. All the measurable HCP were eliminated during the Ab2 CEX whereas 2 ppm remained in the Ab1 eluate (94% removal). Except a small difference in term of non-fragmented IgG, the product quality was quite comparable between Ab1 (99.8% IgG monomer, 93.4% non-fragmented IgG and 48.2% main charged species) and Ab2 (99.5% IgG monomer, 94.6% non-fragmented IgG and 50.8% main charged species) CEX eluates.

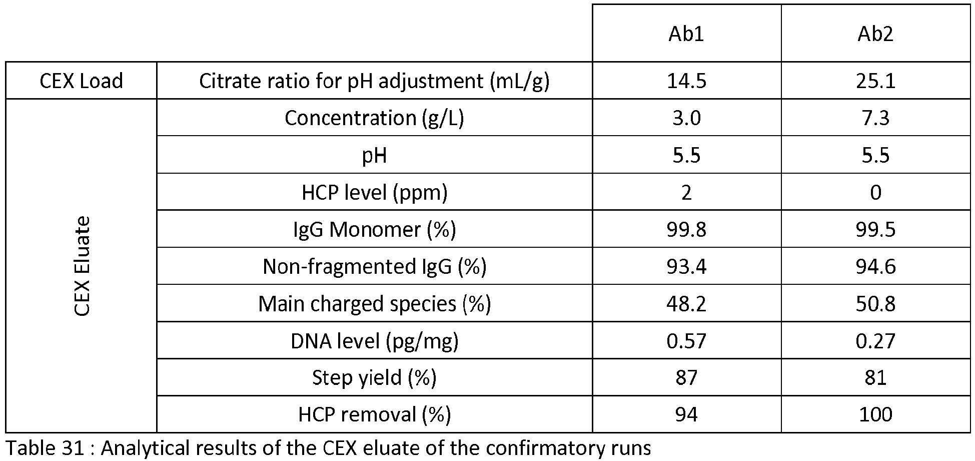

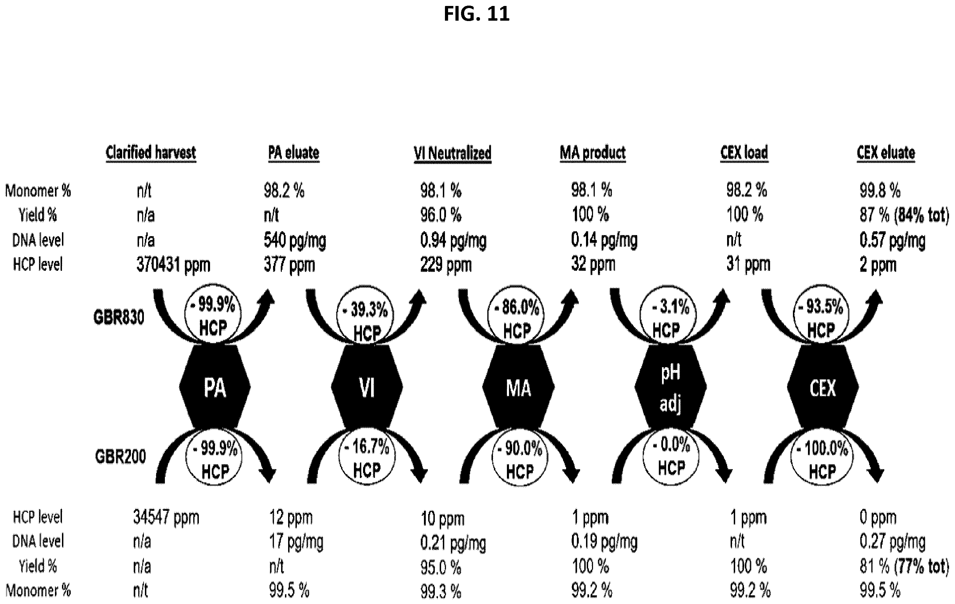

This second polishing step allows to remove some process-related impurities and mAb fragments. Regarding the DNA levels, they were superior to the MA product ones (0.57 pg/mg for Ab1 and 0.27 pg/mg for Ab2) but the variability of the method (20%) could be involved. However, the salt concentration for Ab2 wash step should be developed as the yield has been impacted with the breakthrough of some product. With the applied LF, two CEX runs should be done to process the whole MA product of one run at small scale. This observation showed again how much membrane are efficient in term of LF and flowrates compared to chromatography resins. The comparison of the results obtained with Ab1 and Ab2 is given in Figure 11.

### Conclusions

Compared to RPP, PP2 process optimized by DoE showed its efficiency. The HCP required level was almost reached from the MA step and CEX additionally allowed to remove impurities and to improve the product quality. Almost all the host cell DNA was eliminated after the VI step. In PP2 the removal of UFDF1 step compared to RPP was effected successfully, MA was optimized as a first polishing step post-VI. PP2 platform can process samples faster, due to the increase in MA flowrate and the UFDF 1 removal. Moreover, less buffers could be consumed, and less membrane due to the use of a higher LF.

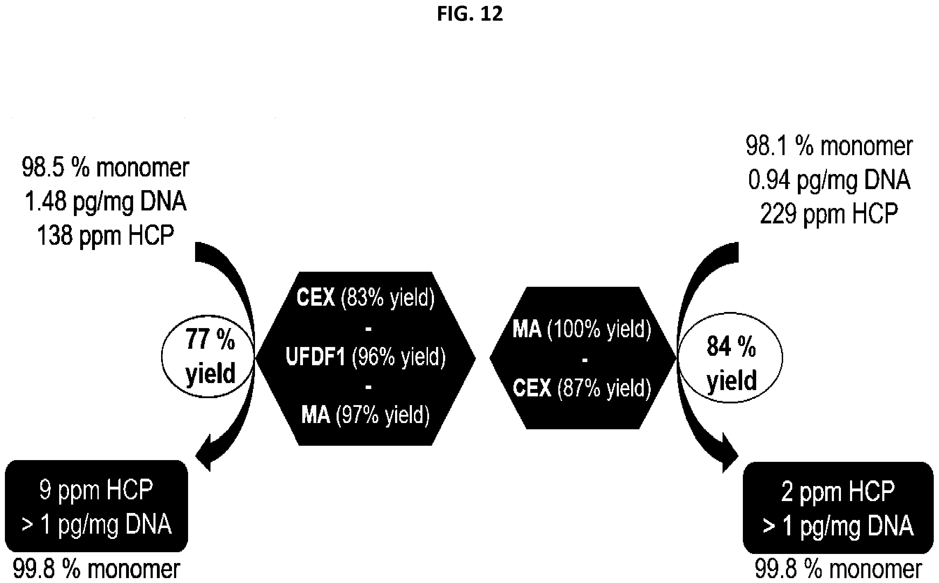

RPP had an overall 77% yield, with 9 ppm of HCP, less than 1 pg/mg of DNA and 99.8% of IgG monomer in the MA product. With the new platform, a 7% higher yield was obtained because of UFDF1 removal and the better CEX step yield (linked to the process scale). The final product quality was comparable with the one obtained with RPP but a better HCP level was reached, with 2 ppm. It is a performance and a quality post-polishing never obtained with antecedent Ab1 process at small, pilot or manufacturing scale Consequently, PP2 is advantageous in respect to RPP (Figure 12).

## Claims

1. A method for preparing a purified therapeutic recombinant antibody solution from a clarified cell harvest, **characterized by** subjecting said clarified cell harvest to steps (i), (ii) and (iii) in the following order: (i) an affinity chromatography step, (ii) a viral inactivation step, (iii) at least two different ion exchange chromatography steps, wherein said purified therapeutic recombinant antibody solution comprises less than 40 ppm of host cell proteins and wherein the yield of said at least two steps of ion exchange chromatography is higher than 70%.

2. The method of any one of the preceding claims wherein said affinity chromatography is protein A affinity chromatography.

3. The method of any one of the preceding claims wherein after the first ion exchange chromatography step, the pH of the resulting ion exchange chromatography eluate is adjusted so as to be the same as the elution buffer of the second ion exchange chromatography step.

4. The method of any one of the preceding claims, wherein said at least two steps of ion exchange chromatography comprise a first step of cation exchange chromatography and a second step of anion exchange chromatography.

5. The method of claims 1 to 3, wherein said at least two steps of ion exchange chromatography comprise a first step of anion exchange chromatography and a second step of cation exchange chromatography.

6. The method of any one of the preceding claims, wherein said cation exchange chromatography is carried out in bind-eluate mode wherein said protein is eluted with an elution buffer comprising between about 20 mM and about 30 mM of Citrate, and between about 50 mM and about 120 mM of NaCl at pH between about 5 and about 6.

7. The method of any one of the preceding claims, wherein said anion exchange chromatography is carried out by membrane absorption in flow-through mode using between about 20 mM and 60 mM of Histidine or Tris buffer at a pH between about 6.5 and about 8.

8. The method of claims 5 to 7, wherein said membrane absorption is carried out using about 50 mM Histidine at a pH between about 7 and about 7.5 at a flow rate comprised between about 2 MV/min and about 30 MV/min, and with a loading factor comprised between about 1 g/mL and about 15 g/mL

9. The method of claim 8, wherein membrane absorption is carried out using about 50 mM Histidine at a pH selected from the group comprising about 7, about 7.2 and about 7.4, at a flow rate selected from the group comprising about 5 MV/min and about 25 MV/min, with a loading factor selected from the group comprising about 2.5 g/mL, about 5 g/mL, about 7.5 g/mL and about 10 g/mL.

10. The method of claim 9, wherein membrane absorption is carried out using about 50 mM Histidine at a pH 7.2, at a flow rate of about 25 MV/min, with a loading factor of about 10 g/mL.

11. The method of any one of claims 5 to 7, wherein said cation exchange chromatography is carried out using an elution buffer comprising about 25 mM of Citrate and about 100 mM or 60 mM of NaCl.

12. The method of claims 8 and 11, wherein the resulting purified therapeutic recombinant antibody solution comprises less than about 20 ppm of host cell proteins and/or less than about 0.8 pg/mg DNA.

13. The method of anyone of the preceding claims, wherein the protein titer of said clarified cell harvest is comprised between about 0.5 g/L and about 10 g/L.

14. A process for preparing a purified therapeutic recombinant antibody solution from a clarified cell harvest, comprising the steps of:
(i) Protein A chromatography of a clarified cell harvested antibody material;
(ii) Incubation of the resulting PA eluate at pH 3.5;
(iii) Neutralization of the resulting viral inactivated solution to pH7.2, followed by 0.2 µm filtration;
(iv) Anion exchange chromatography of the neutralized viral inactivated solution according to claim 10, followed by 0.2 µm filtration;
(v) Cation exchange chromatography of the anion exchange chromatography eluate according to claim 11, followed by 0.2 µm filtration.

15. A process for the production of a bulk drug substance comprising the steps of:
(i) Protein A chromatography of a clarified cell harvested antibody material;
(ii) Incubation of the resulting PA eluate at pH 3.5;
(iii) Neutralization of the resulting viral inactivated solution to pH7.2, followed by 0.2 µm filtration;
(iv) Anion exchange chromatography of the neutralized viral inactivated solution according to claim 10, followed by 0.2 µm filtration;
(v) Cation exchange chromatography of the anion exchange chromatography eluate according to claim 11, followed by 0.2 µm filtration;
(vi) Virus nanofiltration;
(vii) Concentration of the product by ultrafiltration and continuous diafiltration into preformulation buffer, followed by 0.2 um filtration;
(viii) Excipient addition;
(ix) 0.2 pim filtration.

16. A purified therapeutic recombinant antibody solution or bulk drug substance obtained by the process of claim 14 or 15.
